(19) Europäisches Patentamt
European Patent Office
Office européen des brevets

(11) EP 4 238 973 A1

(12) EUROPEAN PATENT APPLICATION

(43) Date of publication:
**06.09.2023 Bulletin 2023/36**

(21) Application number: **22160304.6**

(22) Date of filing: **04.03.2022**

(51) International Patent Classification (IPC):
***C07D 417/12*** (2006.01) ***C07D 285/08*** (2006.01)
***A01N 43/82*** (2006.01)

(52) Cooperative Patent Classification (CPC):
**C07D 417/12; A01N 43/82; A01P 17/00; C07D 285/08**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer AG**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **SUBSTITUTED 1,2,4-THIADIAZOLYL ISONICOTINAMIDES, SALTS OR N-OXIDES THEREOF AND THEIR USE AS HERBICIDALLY ACTIVE SUBSTANCES**

(57) The present invention relates to substituted 1,2,4-thiadiazolyl isonicotinamide of the general formula (I) or salts thereof,

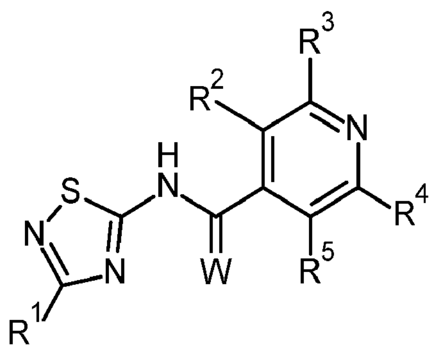

(I)

where the radicals in the general formula (I) correspond to the definitions given in the description, and to their use as herbicides, in particular for controlling broad-leaved weeds and/or weed grasses in crops of useful plants and/or as plant growth regulators for influencing the growth of crops of useful plants.

EP 4 238 973 A1

## Description

The invention relates to the technical field of herbicides and/or plant growth regulators. Specifically, the invention primarily relates to substituted 1,2,4-thiadiazolyl isonicotinamides, and compositions comprising said substituted 1,2,4-thiadiazolyl isonicotinamides. In addition, the present invention relates to processes for the preparation of said substituted 1,2,4-thiadiazolyl isonicotinamides and their use as herbicides and/or plant growth regulators.

In their application, crop protection agents that are known to date for the selective control of harmful plants in crops of useful plants or active compounds for controlling unwanted vegetation sometimes have disadvantages, be it (a) that they have no or insufficient herbicidal activity against particularly harmful plants, (b) that the spectrum of harmful plants which can be controlled with an active compound is not broad enough, (c) that their selectivity in crops of useful plants is too low and/or (d) that they have a toxicologically unfavourable profile.

Furthermore, some active compounds which can be used as plant growth regulators for a number of useful plants cause an unwanted reduction of harvest yields in other useful plants or are not compatible with the crop plant, or only within a narrow application rate range. Additionally, some of the known active compounds cannot be produced economically on an industrial scale owing to precursors and reagents which are difficult to obtain, or that they have only insufficient chemical stabilities.

The prior art discloses several substituted 1,2,4-thiadiazoles that display beneficial properties and uses. The patents US 4,416,683, US 4,515,625, US 4,636,243 and US 4,801,718 relate to heterocyclic substituted N-benzamides and their use as herbicides for controlling unwanted plants.

US 4,343,945 describes that trichloromethyl substituted 1,2,4-thiadiazoles are useful as herbicides, fungicides and insecticides. In addition, DE 2154852 discloses that 5-substituted amino-3-isopropyl-1,2,4-thiadiazoles are efficient pesticides. Furthermore, DE 19601139 concerns the preparation of acylated 5-amino-1,2,4-thiadiazoles and their use as pesticides.

Several documents such as WO 01/36415, WO 01/40206, WO 01/40223 and WO 01/46165 describe that substituted 1,2,4-thiadiazoles are suitable for controlling a variety of pests of domestic animals and livestock.

WO 2017/005717 and WO 2018/108791 also showed that aryl and heteroaryl substituted 1,2,4-thiadiazoles are very effective at controlling unwanted insects in crops of useful plants.

However, substituted 1,2,4-thiadiazolyl isonicotinamides, salts or N-oxides thereof bearing ortho-substituents have not been previously reported. Surprisingly, it has now been found that substituted 1,2,4-thiadiazolyl isonicotinamides, salts or N-oxides thereof bearing ortho-substituents are particularly suitable as herbicides.

In their application, herbicides that are known to date for controlling harmful plants in crops of useful plants or herbicides for controlling unwanted vegetation sometimes have disadvantages, be it (a) that they have no or insufficient herbicidal activity against particularly harmful plants, (b) that the spectrum of harmful plants which can be controlled with an active compound is not broad enough, and/or (c) that the selectivity of the herbicides in and their compatibility with crop plants is too low, thereby causing unwanted damage and/or unwanted reduced harvest yields of the crops.

Thus, there is still a need for alternative herbicides, in particular highly active herbicides that are useful at low application rates and/or having good compatibility with crop plants, for the selective application in plant crops or use on non-crop land. It is also desirable to provide alternative chemical active compounds which may be used in an advantageous manner as herbicides or plant growth regulators.

It is therefore an objective of the present invention to provide compounds having herbicidal activity which are highly effective against economically important harmful plants even at relatively low application rates and that can be used selectively in one or more crop plants.

It has now been found that the compounds of the following formula (I), salts or N-oxides thereof meet said objective(s).

The present invention accordingly provides substituted 1,2,4-thiadiazolyl isonicotinamides of the general formula (I), salts or N-oxides thereof,

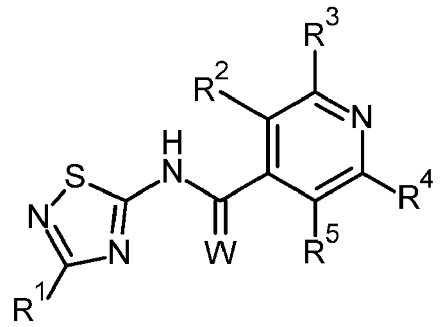

(I)

in which

W represents oxygen or sulfur,

$R^1$ represents hydrogen or halogen,

$R^2$ represents halogen, nitro, $(C_1-C_4)$-alkyl, $(C_1-C_4)$-haloalkyl, $(C_2-C_4)$-alkenyl, $(C_2-C_4)$-haloalkenyl, $(C_1-C_4)$-haloalkyl-$(C_2-C_4)$-alkenyl, $(C_1-C_4)$-alkoxy, $(C_1-C_4)$-haloalkoxy, $(C_1-C_4)$-alkylthio, $(C_1-C_4)$-haloalkylthio, $(C_1-C_4)$-alkylsulfinyl, $(C_1-C_4)$-haloalkylsulfinyl, $(C_1-C_4)$-alkylsulfonyl, $(C_1-C_4)$-haloalkylsulfonyl, $(C_3-C_6)$-cycloalkyl, $(C_3-C_6)$-halocycloalkyl, $(C_1-C_4)$-alkyl-$(C_3-C_6)$-cycloalkyl, $(C_1-C_4)$-alkyl-$(C_3-C_6)$-halocycloalkyl, $(C_1-C_4)$-haloalkyl-$(C_3-C_6)$-cycloalkyl or $(C_1-C_4)$-haloalkyl-$( C_3-C_6)$-halocycloalkyl,

$R^3$ represents hydrogen, halogen, nitro, cyano, $(C_1-C_4)$-alkyl, $(C_1-C_4)$-haloalkyl, $(C_1-C_4)$-alkoxy, $(C_1-C_4)$-haloalkoxy, $(C_3-C_6)$-cycloalkyl or $(C_3-C_6)$-halocycloalkyl,

$R^4$ represents hydrogen, halogen, nitro, cyano, $(C_1-C_4)$-alkyl, $(C_1-C_4)$-haloalkyl, $(C_1-C_4)$-alkoxy, $(C_1-C_4)$-haloalkoxy, $(C_3-C_6)$-cycloalkyl or $(C_3-C_6)$-halocycloalkyl, and

$R^5$ represents hydrogen, halogen, nitro, cyano, $(C_1-C_4)$-alkyl, $(C_1-C_4)$-haloalkyl, $(C_1-C_4)$-alkoxy, $(C_1-C_4)$-haloalkoxy, $(C_3-C_6)$-cycloalkyl or $(C_3-C_6)$-halocycloalkyl.

The compounds of the general formula (I) can form salts by addition of a suitable inorganic or organic acid, for example mineral acids, for example HCl, HBr, $H_2SO_4$, $H_3PO_4$ or $HNO_3$, or organic acids, for example carboxylic acids such as formic acid, acetic acid, propionic acid, oxalic acid, lactic acid or salicylic acid or sulfonic acids, for example p-toluenesulfonic acid, onto a basic group, for example amino, alkylamino, dialkylamino, piperidino, morpholino or pyridino. In such a case, these salts will comprise the conjugated base of the acid as the anion. Suitable substituents in deprotonated form, for example sulfonic acids, particular sulfonamides or carboxylic acids, are capable of forming internal salts with groups, such as amino groups, which are themselves protonatable. Salts may also be formed by action of a base on compounds of the general formula (I). Suitable bases are, for example, organic amines such as trialkylamines, morpholine, piperidine and pyridine, and the hydroxides, carbonates and bicarbonates of ammonium, alkali metals or alkaline earth metals, especially sodium hydroxide, potassium hydroxide, sodium carbonate, potassium carbonate, sodium bicarbonate and potassium bicarbonate. These salts are compounds in which the acidic hydrogen is replaced by an agriculturally suitable cation, for example metal salts, especially alkali metal salts or alkaline earth metal salts, in particular sodium and potassium salts, or else ammonium salts, salts with organic amines or quaternary ammonium salts, for example with cations of the formula $[NR^aR^bR^cR^d]^+$ in which $R^a$ to $R^d$ are each independently an organic radical, especially alkyl, aryl, arylalkyl or alkylaryl. Also suitable are alkylsulfonium and alkylsulfoxonium salts, such as $(C_1-C_4)$-trialkylsulfonium and $(C_1-C_4)$-trialkylsulfoxonium salts.

The substituted 1,2,4-thiadiazolyl isonicotinamides of the general formula (I) according to the invention can, depending on external conditions such as pH, solvent and temperature, be present in various tautomeric structures, all of which are embraced by the general formula (I).

The compounds of the formula (I) used in accordance with the invention and salts or N-oxides thereof are also referred to hereinafter as "compounds of the general formula (I)".

The invention preferably provides compounds of the general formula (I), salts or N-oxides thereof in which

W represents oxygen or sulfur,

$R^1$ represents hydrogen or halogen,

$R^2$ represents halogen, nitro, $(C_1\text{-}C_4)$-alkyl, $(C_1\text{-}C_4)$-haloalkyl, $(C_2\text{-}C_4)$-alkenyl, $(C_2\text{-}C_4)$-haloalkenyl, $(C_1\text{-}C_4)$-alkoxy, $(C_1\text{-}C_4)$-haloalkoxy, $(C_1\text{-}C_4)$-alkylthio, $(C_1\text{-}C_4)$-haloalkylthio, $(C_1\text{-}C_4)$-alkylsulfinyl, $(C_1\text{-}C_4)$-haloalkylsulfinyl, $(C_1\text{-}C_4)$-alkylsulfonyl, $(C_1\text{-}C_4)$-haloalkylsulfonyl, $(C_3\text{-}C_6)$-cycloalkyl, $(C_3\text{-}C_6)$-halocycloalkyl, $(C_1\text{-}C_4)$-alkyl-$(C_3\text{-}C_6)$-cycloalkyl or $(C_1\text{-}C_4)$-alkyl-$(C_3\text{-}C_6)$-halocycloalkyl,

$R^3$ represents hydrogen, halogen, $(C_1\text{-}C_4)$-alkyl, $(C_1\text{-}C_4)$-haloalkyl, $(C_1\text{-}C_4)$-alkoxy or $(C_1\text{-}C_4)$-haloalkoxy,

$R^4$ represents hydrogen, halogen, cyano, $(C_1\text{-}C_4)$-alkyl, $(C_1\text{-}C_4)$-haloalkyl, $(C_1\text{-}C_4)$-alkoxy, $(C_1\text{-}C_4)$-haloalkoxy or $(C_3\text{-}C_6)$-cycloalkyl, and

$R^5$ represents hydrogen, halogen, $(C_1\text{-}C_4)$-alkyl, $(C_1\text{-}C_4)$-haloalkyl, $(C_1\text{-}C_4)$-alkoxy or $(C_1\text{-}C_4)$-haloalkoxy.

The invention more preferably provides compounds of the general formula (I), salts or N-oxides thereof in which

W represents oxygen or sulfur, preferably oxygen,

$R^1$ represents hydrogen or halogen,

$R^2$ represents halogen, nitro, $(C_1\text{-}C_4)$-alkyl, $(C_1\text{-}C_4)$-haloalkyl, $(C_2\text{-}C_4)$-alkenyl, $(C_1\text{-}C_4)$-alkoxy, $(C_1\text{-}C_4)$-haloalkoxy, $(C_1\text{-}C_4)$-alkylthio, $(C_1\text{-}C_4)$-haloalkylthio, $(C_1\text{-}C_4)$-alkylsulfonyl, $(C_1\text{-}C_4)$-haloalkylsulfonyl, $(C_3\text{-}C_6)$-cycloalkyl or $(C_3\text{-}C_6)$-halocycloalkyl,

$R^3$ represents hydrogen, halogen, $(C_1\text{-}C_4)$-alkyl or $(C_1\text{-}C_4)$-haloalkyl,

$R^4$ represents hydrogen, halogen, $(C_1\text{-}C_4)$-alkyl, $(C_1\text{-}C_4)$-haloalkyl, $(C_1\text{-}C_4)$-alkoxy or $(C_1\text{-}C_4)$-haloalkoxy, and

$R^5$ represents hydrogen, halogen, $(C_1\text{-}C_4)$-alkyl or $(C_1\text{-}C_4)$-haloalkyl.

The invention particularly provides compounds of the general formula (I), salts or N-oxides thereof in which

W represents oxygen,

$R^1$ represents hydrogen or halogen,

$R^2$ represents halogen, nitro, $(C_1\text{-}C_4)$-alkyl, $(C_1\text{-}C_4)$-haloalkyl, $(C_1\text{-}C_4)$-haloalkoxy, $(C_1\text{-}C_4)$-alkylthio, $(C_1\text{-}C_4)$-haloalkylthio, $(C_1\text{-}C_4)$-alkylsulfonyl or $(C_3\text{-}C_6)$-cycloalkyl,

$R^3$ represents hydrogen or halogen,

$R^4$ represents hydrogen, halogen, $(C_1\text{-}C_4)$-haloalkyl or $(C_1\text{-}C_4)$-alkoxy, and

$R^5$ represents hydrogen or halogen.

The invention more particularly provides compounds of the general formula (I), salts or N-oxides thereof in which

W represents oxygen,

$R^1$ represents hydrogen, chlorine or bromine,

$R^2$ represents fluorine, chlorine, bromine, iodine, nitro, methyl, trifluoromethyl, trifluoromethoxy, ethylthio, 2,2,2-trifluoroethylthio, ethylsulfonyl or cyclopropyl,

$R^3$ represents hydrogen, fluorine, chlorine or bromine,

$R^4$ represents hydrogen, fluorine, chlorine, bromine, trifluoromethyl or methoxy, and

$R^5$ represents hydrogen, fluorine, chlorine or bromine.

The invention especially provides compounds of the general formula (I), salts or N-oxides thereof in which

W represents oxygen,

$R^1$ represents hydrogen, chlorine or bromine,

$R^2$ represents fluorine, chlorine, bromine, iodine, nitro, trifluoromethyl, trifluoromethoxy, ethylsulfonyl or cyclopropyl,

$R^3$ represents hydrogen, fluorine, chlorine or bromine,

$R^4$ represents hydrogen, fluorine, chlorine, bromine or trifluoromethyl, and

$R^5$ represents hydrogen or bromine.

The definitions of radicals listed above in general terms or within areas of preference apply both to the end products of the general formula (I) and correspondingly to the starting materials or intermediates required for preparation in each case. These radical definitions can be combined with one another as desired, i.e. including combinations between the given preferred ranges.

Primarily for reasons of higher herbicidal activity, better selectivity and/or better producibility, compounds of the abovementioned general formula (I) according to the invention and/or their salts and N-oxides or their use according to the invention are of particular interest in which individual radicals have one of the preferred meanings already specified or specified below, or in particular those in which one or more of the preferred meanings already specified or specified below occur in combination.

With regard to the compounds according to the invention, the terms used above and further below will be elucidated. These are familiar to the person skilled in the art and especially have the definitions elucidated hereinafter: Unless defined differently, names of chemical groups are generally to be understood such that attachment to the skeleton or the remainder of the molecule is via the structural element mentioned last, i.e. for example in the case of ($C_2$-$C_8$)-alkenyloxy via the oxygen atom and in the case of ($C_1$-$C_8$)-alkoxy-($C_1$-$C_4$)-alkyl or ($C_1$-$C_8$)-alkoxycarbonyl-($C_1$-$C_8$)-alkyl, in each case via the carbon atom of the alkyl group.

According to the invention, "alkylsulfonyl" - alone or as part of a chemical group - refers to straight-chain or branched alkylsulfonyl, preferably having 1 to 8 or 1 to 6 carbon atoms, for example (but not limited to) ($C_1$-$C_6$)-alkylsulfonyl such as methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl.

According to the invention, "alkylthio" - alone or as part of a chemical group - denotes straight-chain or branched S-alkyl, preferably having 1 to 8 or 1 to 6 carbon atoms, such as ($C_1$-$C_{10}$)-, ($C_1$-$C_6$)- or ($C_1$-$C_4$)-alkylthio, for example (but not limited to) ($C_1$-$C_6$)-alkylthio such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1-ethyl-2-methylpropylthio.

According to the invention, "alkylsulfinyl (alkyl-S(=O)-)", unless defined differently elsewhere, denotes alkyl radicals which are attached to the skeleton via -S(=O)-, such as ($C_1$-$C_{10}$)-, ($C_1$-$C_6$)- or ($C_1$-$C_4$)-alkylsulfinyl, for example (but not limited to) ($C_1$-$C_6$)-alkylsulfinyl such as methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl,

1,2,2-trimethylpropylsulfinyl, 1-ethyl-1-methylpropylsulfinyl and 1-ethyl-2-methylpropylsulfinyl.

"Alkoxy" denotes an alkyl radical bonded via an oxygen atom, for example (but not limited to) ($C_1$-$C_6$)-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. Alkenyloxy denotes an alkenyl radical attached via an oxygen atom, and alkynyloxy denotes an alkynyl radical attached via an oxygen atom, such as ($C_2$-$C_{10}$)-, ($C_2$-$C_6$)- or ($C_2$-$C_4$)-alkenoxy and ($C_3$-$C_{10}$)-, ($C_3$-$C_6$)- or ($C_3$-$C_4$)-alkynoxy.

The term "halogen" denotes, for example, fluorine, chlorine, bromine or iodine. If the term is used for a radical, "halogen" denotes, for example, a fluorine, chlorine, bromine or iodine atom.

According to the invention, "alkyl" denotes a straight-chain or branched open-chain, saturated hydrocarbon radical which is optionally mono- or polysubstituted, and in the latter case is referred to as "substituted alkyl". Preferred substituents are halogen atoms, alkoxy, haloalkoxy, cyano, alkylthio, haloalkylthio, amino or nitro groups, particular preference being given to methoxy, methyl, fluoroalkyl, cyano, nitro, fluorine, chlorine, bromine or iodine.

The prefix "di" includes the combination of equal or different alkyl radicals, e.g. dimethyl or methyl(ethyl) or ethyl(methyl).

"Haloalkyl", "-alkenyl" and "-alkynyl" respectively denote alkyl, alkenyl and alkynyl partially or fully substituted by identical or different halogen atoms, for example monohaloalkyl such as $CH_2CH_2Cl$, $CH_2CH_2Br$, $CHClCH_3$, $CH_2Cl$, $CH_2F$; perhaloalkyl such as $CCl_3$, $CClF_2$, $CFCl_2$,$CF_2CClF_2$, $CF_2CClFCF_3$; polyhaloalkyl such as $CH_2CHFCl$, $CF_2CClFH$, $CF_2CBrFH$, $CH_2CF_3$; the term perhaloalkyl also encompasses the term perfluoroalkyl.

"Haloalkoxy" is, for example, $OCF_3$, $OCHF_2$, $OCH_2F$, $OCF_2CF_3$, $OCH_2CF_3$ and $OCH_2CH_2Cl$; this applies correspondingly to haloalkenyl and other halogen-substituted radicals.

The expression "($C_1$-$C_4$)-alkyl" mentioned here by way of example is a brief notation for straight-chain or branched alkyl having one to 4 carbon atoms according to the range stated for carbon atoms, i.e. encompasses the methyl, ethyl, 1-propyl, 2-propyl, 1-butyl, 2-butyl, 2-methylpropyl or tert-butyl radicals. General alkyl radicals with a larger specified range of carbon atoms, e.g. "($C_1$-$C_6$)-alkyl", correspondingly also encompass straight-chain or branched alkyl radicals with a greater number of carbon atoms, i.e. according to the example also the alkyl radicals having 5 and 6 carbon atoms.

Unless stated specifically, preference is given to the lower carbon skeletons, for example having from 1 to 6 carbon atoms, or having from 2 to 6 carbon atoms in the case of unsaturated groups, in the case of the hydrocarbyl radicals such as alkyl, alkenyl and alkynyl radicals, including in composite radicals. Alkyl radicals, including in composite radicals such as alkoxy, haloalkyl, etc., are, for example, methyl, ethyl, n-propyl or i-propyl, n-, i-, t- or 2-butyl, pentyls, hexyls such as n-hexyl, i-hexyl and 1,3-dimethylbutyl, heptyls such as n-heptyl, 1-methylhexyl and 1,4-dimethylpentyl; alkenyl and alkynyl radicals are defined as the possible unsaturated radicals corresponding to the alkyl radicals, where at least one double bond or triple bond is present. Preference is given to radicals having one double bond or triple bond.

The term "alkenyl" also includes, in particular, straight-chain or branched open-chain hydrocarbon radicals having more than one double bond, such as 1,3-butadienyl and 1,4-pentadienyl, but also allenyl or cumulenyl radicals having one or more cumulated double bonds, for example allenyl (1,2-propadienyl), 1,2-butadienyl and 1,2,3-pentatrienyl. Alkenyl denotes, for example, vinyl which may optionally be substituted by further alkyl radicals, for example (but not limited thereto) ($C_2$-$C_6$)-alkenyl such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "cycloalkyl" denotes a carbocyclic saturated ring system having preferably 3-8 ring carbon atoms, for example cyclopropyl, cyclobutyl, cyclopentyl or cyclohexyl, which optionally has further substitution, preferably by hydrogen, alkyl, alkoxy, cyano, nitro, alkylthio, haloalkylthio, halogen, alkenyl, alkynyl, haloalkyl, amino, alkylamino, dialkylamino, alkoxycarbonyl, hydroxycarbonyl, arylalkoxycarbonyl, aminocarbonyl, alkylaminocarbonyl, cycloalkylaminocarbonyl. In the case of optionally substituted cycloalkyl, cyclic systems with substituents are included, also including

substituents with a double bond on the cycloalkyl radical, for example an alkylidene group such as methylidene. In the case of optionally substituted cycloalkyl, polycyclic aliphatic systems are also included, for example bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl and adamantan-2-yl, but also systems such as 1,1'-bi(cyclopropyl)-1-yl, 1,1'-bi(cyclopropyl)-2-yl, for example. The term "($C_3$-$C_7$)-cycloalkyl" is a brief notation for cycloalkyl having three to 7 carbon atoms, corresponding to the range specified for carbon atoms.

In the case of substituted cycloalkyl, spirocyclic aliphatic systems are also included, for example spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl.

According to the invention, "haloalkylthio" - on its own or as constituent part of a chemical group - represents straight-chain or branched S-haloalkyl, preferably having 1 to 8, or having 1 to 6 carbon atoms, such as ($C_1$-$C_8$)-, ($C_1$-$C_6$)- or ($C_1$-$C_4$)-haloalkylthio, for example (but not limited thereto) trifluoromethylthio, pentafluoroethylthio, difluoromethyl, 2,2-difluoroeth-1-ylthio, 2,2,2-difluoroeth-1-ylthio, 3,3,3-prop-1-ylthio.

"Halocycloalkyl" denotes a cycloalkyl, which is partially or fully substituted by identical or different halogen atoms, such as F, Cl and Br, or by haloalkyl, such as trifluoromethyl or difluoromethyl, for example 1-fluorocycloprop-1-yl, 2-fluorocycloprop-1-yl, 2,2-difluorocycloprop-1-yl, 1-fluorocyclobut-1-yl, 1-trifluoromethylcycloprop-1-yl, 2-trifluoromethylcycloprop-1-yl, 1-chlorocycloprop-1-yl, 2-chlorocycloprop-1-yl, 2,2-dichlorocycloprop-1-yl, 3,3-difluorocyclobutyl.

If the compounds can form, through a hydrogen shift, tautomers whose structure is not formally covered by the general formula (I), these tautomers are nevertheless covered by the definition of the inventive compounds of the general formula (I), unless a particular tautomer is under consideration. For example, many carbonyl compounds may be present both in the keto form and in the enol form, both forms being encompassed by the definition of the compound of the general formula (I).

Depending on the nature of the substituents and the manner in which they are attached, the compounds of the general formula (I) may be present as stereoisomers. The general formula (I) embraces all possible stereoisomers defined by the specific three-dimensional form thereof, such as enantiomers, diastereomers, Z and E isomers. If, for example, one or more alkenyl groups are present, diastereomers (Z and E isomers) may occur. If, for example, one or more asymmetric carbon atoms are present, enantiomers and diastereomers may occur. Stereoisomers can be obtained from the mixtures obtained in the preparation by customary separation methods. The chromatographic separation can be affected either on the analytical scale to find the enantiomeric excess or the diastereomeric excess, or else on the preparative scale to produce test specimens for biological testing. It is likewise possible to selectively prepare stereoisomers by using stereoselective reactions with use of optically active starting materials and/or auxiliaries. The invention thus also relates to all stereoisomers which are embraced by the general formula (I) but are not shown in their specific stereomeric form, and to mixtures thereof.

If the compounds are obtained as solids, the purification can also be carried out by recrystallization or digestion. If individual compounds of general formula (I) cannot be obtained in a satisfactory manner by the routes described below, they can be prepared by derivatization of other compounds of general formula (I).

Suitable isolation methods, purification methods and methods for separating stereoisomers of compounds of the general formula (I) are methods generally known to the person skilled in the art from analogous cases, for example by physical processes such as crystallization, chromatographic methods, in particular column chromatography and HPLC (high pressure liquid chromatography), distillation, optionally under reduced pressure, extraction and other methods, any mixtures that remain can generally be separated by chromatographic separation, for example on chiral solid phases. Suitable for preparative amounts or on an industrial scale are processes such as crystallization, for example of diastereomeric salts which can be obtained from the diastereomer mixtures using optically active acids and, if appropriate, provided that acidic groups are present, using optically active bases.

Synthesis of substituted 1,2,4-thiadiazolyl isonicotinamides of the general formula (I):

The substituted 1,2,4-thiadiazolyl isonicotinamides of the general formula (I) according to the invention can be prepared using known processes. The synthesis routes used and examined proceed from commercially available or easily synthesised substituted 1,2,4-thiadiazoles, or salts thereof (e.g. hydrochloride or dihydrochloride) and substituted carboxylic acids. In the following schemes, the groups $R^1$, $R^2$, $R^3$, $R^4$, $R^5$ and W of the general formula (I) have the meanings defined above, unless exemplary, but not limiting definitions are given. The first synthesis route for substituted 1,2,4-thiadiazolyl isonicotinamides of the general formula (I) proceeds via an optionally substituted aminothiadiazole (II), or salt thereof and an optionally substituted carboxylic acid (III) (Scheme 1). To this end, a substituted aminothiadiazole is reacted with a substituted carboxylic acid in the presence of a suitable coupling reagent (e.g. thionyl chloride) and a suitable base (e.g. 1-methyl-1H-imidazole) to afford the target 1,2,4-thiadiazolyl isonicotinamides (I) (cf. US2019/0233382). Alternatively, a salt of the aminothiadiazole can be used, with the free amine being generated in the reaction mixture. A plethora of amide coupling reagents and bases exist that could be used to achieve this reaction (cf. Chem. Soc. Rev., 2009, 38, 606-631). Other suitable amide coupling reagents include but are not limited to oxalyl

chloride, T3P (propanephosphonic acid anhydride), DIC (1,3-diisopropylcarbodiimide) and HATU (1-[bis(dimethylamino)methylene]-1H-1,2,3-triazolo[4,5-b]pyridinium 3-oxide hexafluorophosphate) and other suitable bases include but are not limited to triethylamine, DIPEA (N,N-diisopropylethylamine) and pyridine. In Scheme 1 below, $R^1$, $R^2$, $R^3$, $R^4$ and $R^5$ have the meanings defined above and W = oxygen.

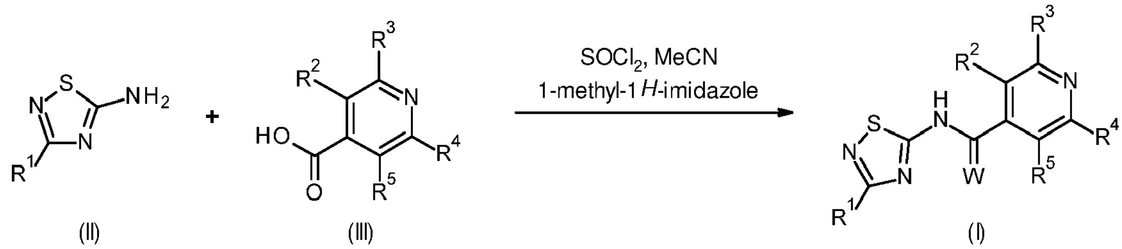

(II) (III) (I)

Scheme 1.

The synthesis of substituted 1,2,4-thiadiazolyl isonicotinamides of the general formula (I) can alternatively be completed via direct amide coupling of an optionally substituted acid chloride (IV) with an optionally substituted 1,2,4-thiadiazole (II) (Scheme 2). Following this method, acid chloride (IV) which has been previously prepared from the corresponding carboxylic acid (III) using a chlorinating reagent (e.g. oxalyl chloride with catalytic amounts of DMF = N,N-dimethylformamide), is reacted with 1,2,4-thiadiazole (II) in the presence of a base (e.g. triethylamine) and a suitable solvent (e.g. THF = tetrahydrofuran) to afford 1,2,4-thiadiazolyl isonicotinamide (I). A wide variety of reagents can be used for this transformation (cf. Chem. Soc. Rev., 2009, 38, 606-631) as well as utilising an appropriate catalyst (e.g. DMAP = 4-dimethylaminopyridine). In Scheme 2 below, $R^1$, $R^2$, $R^3$, $R^4$ and $R^5$ have the meanings defined above and W = oxygen.

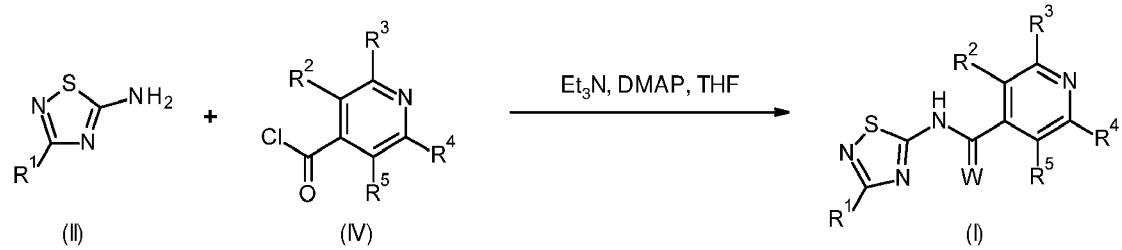

(II) (IV) (I)

Scheme 2.

The synthesis of substituted 1,2,4-thiadiazolyl isonicotinamides of the general formula (I) can, in addition, be achieved by the direct amide coupling of a substituted 1,2,4-thiadiazole (II) with an optionally substituted ester (V) (Scheme 3). For these reactions to proceed an appropriate reagent (e.g. trimethylaluminium) and a suitable solvent (e.g. dichloromethane or toluene) are necessary (cf. Chem. Commun., 2008, 1100-1102). This transformation can also be accomplished using a variety of other reagents that are known in the literature (cf. Tetrahedron Lett., 2006, 47, 5767-5769). In Scheme 3 below, $R^1$, $R^2$, $R^3$, $R^4$ and $R^5$ have the meanings defined above and W = oxygen.

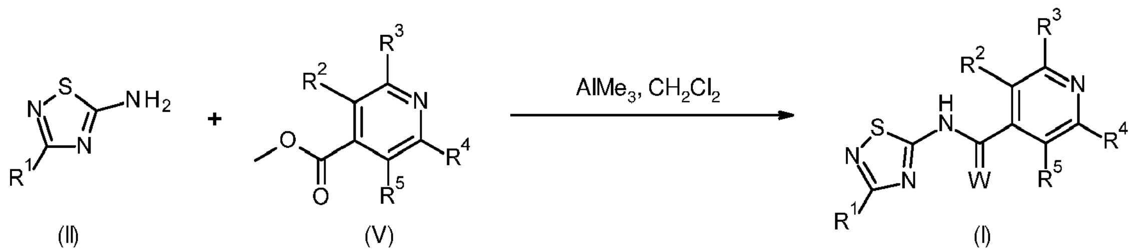

(II) (V) (I)

Scheme 3.

The synthesis of substituted 1,2,4-thiadiazolyl isonicotinamides of the general formula (I) can also be accomplished by functionalizing an independently prepared compound of the general formula (I). Following this method (Scheme 4), 1,2,4-thiadiazolyl isonicotinamide (Ia) is coupled with a substituted zinc reagent (VI) in a Negishi cross-coupling

reaction using an appropriate palladium complex (e.g. Pd($t$Bu$_3$P)$_2$) in a suitable solvent (e.g. THF = tetrahydrofuran) to afford the target 1,2,4-thiadiazolyl isonicotinamides of general formula (I). In Scheme 4 below $R^1$, $R^2$, $R^3$, $R^4$ and $R^5$ have the meanings defined above, W = oxygen and X = chlorine, bromine or iodine.

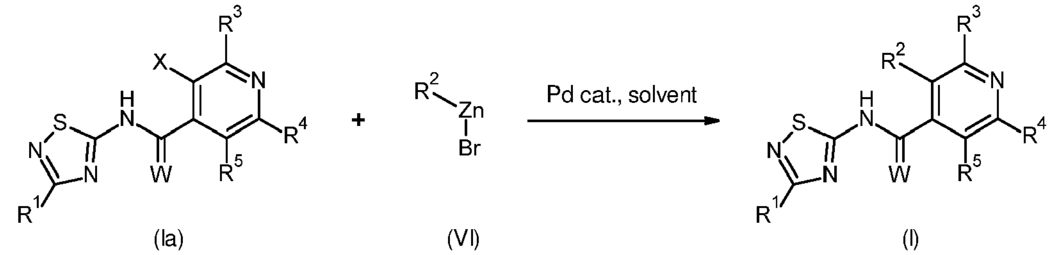

(Ia) (VI) (I)

Scheme 4.

The synthesis of substituted 1,2,4-thiadiazolyl nicotinamides of the general formula (I) can furthermore be completed using other known cross-coupling reactions to prepare the target compounds of the general formula (I). Such examples include, but are not limited to, the Heck reaction (cf. J. Org. Chem., 2011, 76, 8036-8041), Hiyama cross-coupling (cf. J. Am. Chem. Soc., 2003, 125, 5616-5617), Stille cross-coupling (cf. Angew. Chem. Int. Ed., 2004, 43, 1132-1136) and the Suzuki-Miyaura cross-coupling (cf. WO 2015/123133).

Selected detailed synthesis examples for the compounds of the general formula (I) according to the invention are given below. The example numbers mentioned correspond to the numbering scheme in Schemes 1 to 4 and Table 1. The $^1$H-NMR spectroscopy data reported for the chemical examples described in the sections that follow were obtained on Bruker instruments at 600 MHz, 400 MHz or 300 MHz using $CDCl_3$ or $d_6$-DMSO as the solvent with tetramethylsilane ($\delta$ = 0.00 ppm) as the internal standard. The signals listed have the meanings given below: br = broad; s = singlet, d = doublet, t = triplet, dd = doublet of doublets, ddd = doublet of a doublet of doublets, m = multiplet, q = quartet, qu = quintet, sext = sextet, sept = septet, dq = doublet of quartets, dt = doublet of triplets.

Synthesis examples:

No. I-012: 2-chloro-N-(1,2,4-thiadiazol-5-yl)-5-(trifluoromethoxy)pyridine-4-carboxamide

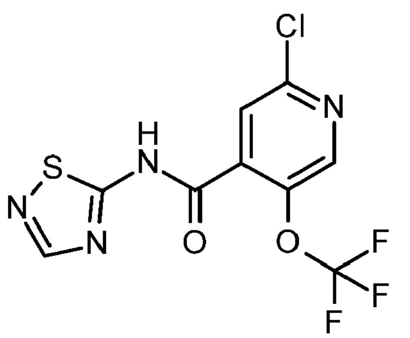

To a stirred suspension of 2-chloro-5-(trifluoromethoxy)pyridine-4-carboxylic acid (150 mg, 0.621 mmol, 1.0 eq.), 1,2,4-thiadiazol-5-amine (69 mg, 0.683 mmol, 1.1 eq.) and 3 Å molecular sieves in MeCN (10 mL) at RT was added 1-methyl-1H-imidazole (0.24 mL, 2.98 mmol, 4.8 eq.). The resulting mixture was stirred at RT for 20 mins, thionyl chloride (72 $\mu$L, 0.994 mmol, 1.6 eq.) was added and the reaction mixture was heated at 70 °C for 12 h. The reaction mixture was allowed to cool to RT and was then diluted with water and $CH_2Cl_2$. The phases were separated using a phase separation cartridge and the organic phase was concentrated under reduced pressure. The resulting residue was purified by flash column chromatography on silica gel eluting with n-heptane/EtOAc (90:10 $\rightarrow$ 0:100) to afford compound 1-012 (64 mg, 31% yield).

No. I-013: 2-chloro-N-(1,2,4-thiadiazol-5-yl)-5-(trifluoromethyl)pyridine-4-carboxamide

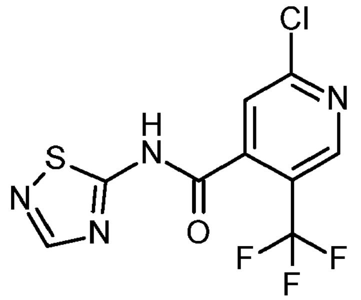

To a stirred suspension of 2-chloro-5-(trifluoromethyl)pyridine-4-carboxylic acid (113 mg, 0.500 mmol, 1.0 eq.), 1,2,4-thiadiazol-5-amine (56 mg, 0.550 mmol, 1.1 eq.) and 3 Å molecular sieves in MeCN (5 mL) at RT was added 1-methyl-1H-imidazole (0.19 mL, 2.40 mmol, 4.8 eq.). The resulting mixture was stirred at RT for 20 mins, thionyl chloride (58 μL, 0.800 mmol, 1.6 eq.) was added and the reaction mixture was heated at 80 °C for 7 h. The reaction mixture was diluted with water and extracted with EtOAc. The organic extract was dried over anhydrous $Na_2SO_4$, filtered and concentrated under reduced pressure. The resulting residue was purified by flash column chromatography on silica gel eluting with n-heptane/EtOAc (100:0 → 0:100) to afford compound 1-013 (102 mg, 65% yield) as a white solid.

No. I-023: N-(3-chloro-1,2,4-thiadiazol-5-yl)-3-iodo-pyridine-4-carboxamide

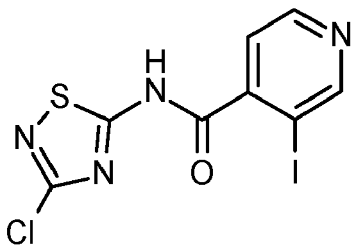

To a stirred suspension of 3-iodopyridine-4-carboxylic acid (200 mg, 0.803 mmol, 1.0 eq.), 3-chloro-1,2,4-thiadiazol-5-amine (152 mg, 1.12 mmol, 1.4 eq.) and 3 Å molecular sieves in MeCN (10 mL) at RT was added 1-methyl-1H-imidazole (0.31 mL, 3.86 mmol, 4.8 eq.). The resulting mixture was stirred at RT for 20 mins, thionyl chloride (123 μL, 1.69 mmol, 2.1 eq.) was added and the reaction mixture was stirred at RT for 48 h. The reaction mixture was diluted with water and extracted with $CH_2Cl_2$. The organic extract was dried over anhydrous $Na_2SO_4$, filtered and concentrated under reduced pressure. The resulting residue was purified by flash column chromatography on silica gel eluting with n-heptane/EtOAc (100:0 → 0:100) to afford compound 1-023 (121 mg, 37% yield).

No. I-027: 5-bromo-2-fluoro-N-(1,2,4-thiadiazol-5-yl)pyridine-4-carboxamide

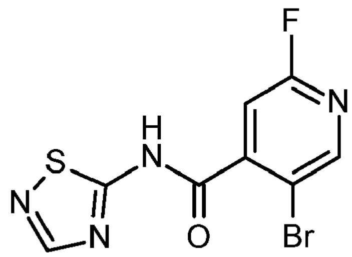

To a stirred suspension of 5-bromo-2-fluoro-pyridine-4-carboxylic acid (150 mg, 0.682 mmol, 1.0 eq.), 1,2,4-thiadiazol-5-amine hydrochloride (103 mg, 0.750 mmol, 1.1 eq.) and 3 Å molecular sieves in MeCN (3 mL) at RT was added 1-methyl-1H-imidazole (0.26 mL, 3.27 mmol, 4.8 eq.). The resulting mixture was stirred at RT for 20 mins, thionyl chloride (80 μL, 1.09 mmol, 1.6 eq.) was added and the reaction mixture was stirred at RT for 60 h. The reaction mixture

was allowed to cool to RT and was then diluted with water and $CH_2Cl_2$. The phases were separated using a phase separation cartridge and the organic phase was concentrated under reduced pressure. To the resulting residue was added a small quantity of $CH_2Cl_2$ and the precipitated solid was collected by filtration to afford compound I-027 (92 mg, 45% yield).

No. I-034: 5-cyclopropyl-2-fluoro-N-(1,2,4-thiadiazol-5-yl)pyridine-4-carboxamide

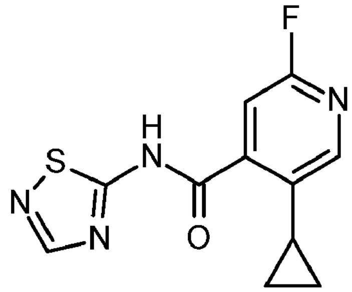

To a stirred mixture of compound I-027 (85 mg, 0.280 mmol, 1.0 eq.) and Pd(*t*$Bu_3P)_2$ (21 mg, 0.042 mmol, 0.15 eq.) in THF (2.5 mL) at 0 °C under an atmosphere of argon was added cyclopropylzinc bromide (2.80 mL, 1.40 mmol, 5.0 eq., 0.5 M solution in THF) dropwise. The resulting mixture was stirred at 0 °C for 1 h. The reaction mixture was quenched with sat.aq. $NH_4Cl$ and extracted with EtOAc. The organic extract was dried over anhydrous $Na_2SO_4$, filtered and concentrated under reduced pressure. The resulting residue was purified by flash column chromatography on silica gel eluting with n-heptane/EtOAc (100:0 → 30:70) to afford compound 1-034 (37 mg, 47% yield).

In analogy to the preparation examples cited above and recited at the appropriate point and taking account of the general details relating to the preparation of substituted 1,2,4-thiadiazolyl isonicotinamides, the compounds cited below are obtained.

Table 1: Examples of preferred compounds of the general formula (I)

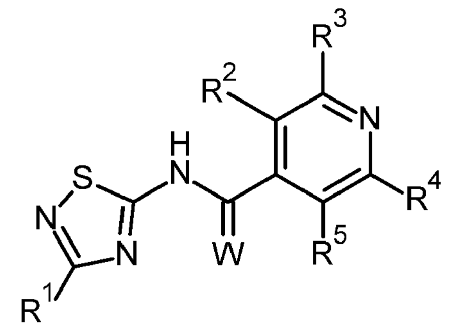

(I)

| Ex. No. | $R^1$ | $R^2$ | $R^3$ | $R^4$ | $R^5$ | W |
|---|---|---|---|---|---|---|
| 1-001 | H | $CF_3$ | H | H | H | O |
| 1-002 | H | Cl | H | H | H | O |
| I-003 | H | Br | H | H | H | O |
| 1-004 | H | I | H | H | H | O |
| 1-005 | H | 2,2,2-trifluoroethylthio | H | H | H | O |
| 1-006 | H | Br | H | $CF_3$ | H | O |
| I-007 | H | Br | H | Cl | H | O |
| I-008 | H | F | H | F | F | O |

(continued)

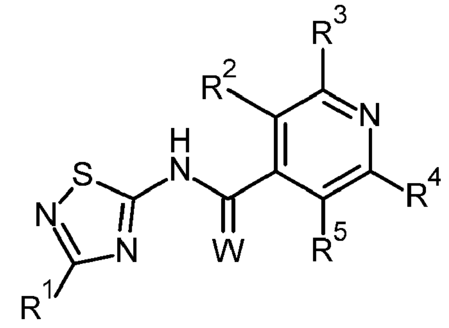

(I)

| Ex. No. | $R^1$ | $R^2$ | $R^3$ | $R^4$ | $R^5$ | W |
|---|---|---|---|---|---|---|
| I-009 | H | Cl | H | H | Cl | O |
| 1-010 | H | F | H | H | F | O |
| I-011 | H | Cl | H | Cl | H | O |
| 1-012 | H | $OCF_3$ | H | Cl | H | O |
| I-013 | H | $CF_3$ | H | Cl | H | O |
| 1-014 | H | Cl | H | Br | Cl | O |
| I-015 | H | Br | H | H | Br | O |
| I-016 | H | F | H | Cl | H | O |
| 1-017 | H | F | Cl | H | H | O |
| I-018 | H | Cl | F | H | H | O |
| I-019 | H | Cl | Cl | H | H | O |
| 1-020 | H | Br | Br | H | H | O |
| 1-021 | H | Br | Cl | H | H | O |
| 1-022 | Br | $CF_3$ | H | Cl | H | O |
| 1-023 | Cl | I | H | H | H | O |
| 1-024 | Br | I | H | H | H | O |
| 1-025 | Cl | $CF_3$ | H | Cl | H | O |
| 1-026 | H | cyclopropyl | H | H | H | O |
| I-027 | H | Br | H | F | H | O |
| 1-028 | H | Me | H | H | H | O |
| I-029 | H | F | H | H | H | O |
| 1-030 | H | F | H | F | H | O |
| 1-031 | H | F | F | H | H | O |
| 1-032 | H | F | F | F | H | O |
| Ex. No. | $R^1$ | $R^2$ | $R^3$ | $R^4$ | $R^5$ | W |
| I-033 | H | F | H | OMe | H | O |
| 1-034 | H | cyclopropyl | H | F | H | O |
| 1-035 | H | cyclopropyl | H | Cl | H | O |
| I-036 | H | Br | H | H | F | O |

(continued)

| Ex. No. | $R^1$ | $R^2$ | $R^3$ | $R^4$ | $R^5$ | W |
|---|---|---|---|---|---|---|
| I-037 | H | Ethylthio | H | H | H | O |
| I-038 | H | Cl | H | Br | H | O |
| I-039 | H | ethyl sulfonyl | H | H | H | O |
| 1-040 | H | $NO_2$ | H | Cl | H | O |
| I-041 | H | Br | H | OMe | H | O |
| 1-042 | H | $NO_2$ | H | H | H | O |

Spectroscopic data of selected table examples:
The spectroscopic data listed hereinafter for selected table examples were evaluated via conventional $^1$H-NMR interpretation or via NMR peak list methods.

a) Conventional $^1$H-NMR interpretation

No. I-002: $^1$H-NMR (400 MHz, $CDCl_3$): $\delta_H$ 11.24 (br s, 1H), 8.86.8.82 (m, 1H), 8.78-8.74 (m, 1H), 8.31 (s, 1H), 7.81-7.78 (m, 1H).
No. 1-005: $^1$H-NMR (400 MHz, $CDCl_3$): $\delta_H$ 11.83 (br s, 1H), 9.03-9.01 (m, 1H), 8.85-8.82 (m, 1H), 8.06 (s, 1H), 7.76-7.73 (m, 1H), 3.61-3.52 (m, 2H).
No. 1-012: $^1$H-NMR (400 MHz, DMSO-$d_6$): $\delta_H$ 13.96 (br s, 1H), 8.82-8.80 (m, 1H), 8.61 (s, 1H), 8.17 (s, 1H).
No. 1-021: $^1$H-NMR (400 MHz, DMSO-$d_6$): $\delta_H$ 13.84 (br s, 1H), 8.63-8.59 (m, 2H), 7.80-7.77 (m, 1H).
No. 1-022: $^1$H-NMR (400 MHz, $CDCl_3$): $\delta_H$ 9.96 (br s, 1H), 8.89 (s, 1H), 7.66 (s, 1H).
No. I-023: $^1$H-NMR (400 MHz, $CDCl_3$): $\delta_H$ 10.31 (br s, 1H), 9.15 (s, 1H), 8.77-8.74 (m, 1H), 7.57-7.53 (m, 1H).
No. I-024: $^1$H-NMR (400 MHz, $CDCl_3$): $\delta_H$ 10.08 (br s, 1H), 9.15 (s, 1H), 8.78-8.74 (m, 1H), 7.57-7.54 (m, 1H).
No. 1-025: $^1$H-NMR (400 MHz, $CDCl_3$): $\delta_H$ 10.93 (br s, 1H), 8.89 (s, 1H), 7.65 (s, 1H).

b) NMR peak list method

$^1$H-NMR data of selected examples are written in form of $^1$H-NMR-peak lists. To each signal peak are listed the $\delta$-value in ppm and the signal intensity in round brackets. Between the $\delta$-value - signal intensity pairs are semicolons as delimiters.
The peak list of an example has therefore the form:
$\delta_1$ (intensity$_1$); $\delta_2$ (intensity$_2$);........; $\delta_i$ (intensity$_i$);......; $\delta_n$, (intensity$_n$)
Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.
For calibrating chemical shift for $^1$H spectra, we use tetramethylsilane and/or the chemical shift of the solvent used, especially in the case of spectra measured in DMSO. Therefore in NMR peak lists, tetramethylsilane peak can occur but not necessarily.
The $^1$H-NMR peak lists are similar to classical $^1$H-NMR prints and contains therefore usually all peaks, which are listed at classical NMR-interpretation.
Additionally, they can show like classical $^1$H-NMR prints signals of solvents, stereoisomers of the target compounds, which are also object of the invention, and/or peaks of impurities.
To show compound signals in the delta-range of solvents and/or water the usual peaks of solvents, for example peaks of DMSO in DMSO-$d_6$ and the peak of water are shown in our $^1$H-NMR peak lists and have usually on average a high intensity.
The peaks of stereoisomers of the target compounds and/or peaks of impurities have usually on average a lower intensity than the peaks of target compounds (for example with a purity >90%).
Such stereoisomers and/or impurities can be typical for the specific preparation process. Therefore, their peaks can help to recognize the reproduction of our preparation process via "side-products-fingerprints".
An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical $^1$H-NMR interpretation.
Further details of NMR-data description with peak lists you find in the publication "Citation of NMR Peaklist

Data within Patent Applications" of the Research Disclosure Database Number 564025.

| |
|---|
| I-001: $^{1}$H-NMR(400.0 MHz, CDCl3):<br>$\delta$= 9.1720 (9.3); 9.0868 (6.1); 9.0745 (6.2); 7.6299 (7.4); 7.6176 (7.3); 7.5771 (16.0); 7.5194 (1.0); 7.2605 (147.5); 7.2286 (0.8); 7.2260 (0.7); 6.9965 (0.8); 3.4851 (0.5); 2.0040 (1.2); 1.5866 (0.9); 0.0080 (2.4); -0.0002 (62.2); -0.0084 (2.7) |
| 1-003: $^{1}$H-NMR(400.0 MHz, CDCl3):<br>$\delta$= 11.4218 (1.2); 8.9697 (16.0); 8.7901 (11.1); 8.7779 (11.7); 8.0341 (12.4); 7.6845 (8.7); 7.6724 (8.4); 7.5188 (1.0); 7.2600 (166.3); 6.9958 (1.0); 5.3000 (1.5); 4.0124 (1.0); 2.0076 (1.3); 1.5723 (1.7); 0.1458 (0.8); 0.0080 (6.2); -0.0002 (218.2); -0.0085 (6.0); -0.1497 (0.8) |
| 1-004: $^{1}$H-NMR(400.0 MHz, CDCl3):<br>$\delta$= 12.5879 (1.4); 9.1807 (16.0); 9.1507 (0.6); 8.7924 (11.0); 8.7803 (11.2); 8.3683 (1.0); 7.6815 (14.7); 7.5328 (9.8); 7.5315 (9.8); 7.5206 (10.2); 7.2612 (171.9); 6.9971 (0.9); 2.0075 (1.6); 1.5769 (2.4); 0.0079 (3.5); -0.0002 (101.7); -0.0085 (2.8) |
| I-006: $^{1}$H-NMR(400.0 MHz, $d_6$-DMSO):<br>$\delta$= 13.8862 (1.1); 9.1676 (8.8); 8.6175 (16.0); 8.4331 (10.5); 3.3247 (6.9); 2.6706 (0.6); 2.5241 (1.1); 2.5195 (1.5); 2.5107 (32.1); 2.5061 (71.8); 2.5016 (101.8); 2.4970 (72.2); 2.4924 (32.8); 2.3284 (0.6); 0.0080 (1.3); -0.0002 (50.4); -0.0085 (1.5) |
| 1-007: $^{1}$H-NMR(400.6 MHz, $d_6$-DMSO):<br>$\delta$= 13.8549 (0.8); 8.8128 (10.4); 8.8115 (10.3); 8.6092 (16.0); 8.0470 (10.9); 8.0457 (10.6); 3.3305 (1.2); 2.5209 (0.6); 2.5121 (9.3); 2.5075 (20.8); 2.5029 (29.7); 2.4983 (20.9); 2.4938 (9.6); 0.0080 (0.5); -0.0002 (18.2); -0.0085 (0.5) |
| 1-008: $^{1}$H-NMR(400.0 MHz, $d_6$-DMSO):<br>$\delta$= 8.6530 (16.0); 8.4489 (5.0); 8.4463 (4.9); 8.4437 (4.2); 2.5347 (1.8); 2.5303 (4.0); 2.5257 (5.6); 2.5212 (4.1); 2.5167 (1.8); - 0.0002 (2.8) |
| 1-009: $^{1}$H-NMR(400.0 MHz, CDCl3):<br>$\delta$= 8.7227 (16.0); 8.5648 (1.8); 7.6343 (4.2); 7.2607 (23.5); 3.2018 (2.7); 1.5629 (0.7); 1.2556 (0.5); 0.0080 (0.8); -0.0002 (31.2); -0.0084 (1.0) |
| 1-010: $^{1}$H-NMR(400.0 MHz, $d_6$-DMSO):<br>$\delta$= 8.8099 (16.0); 8.6346 (6.9); 2.5159 (4.0); 2.5114 (8.9); 2.5068 (12.5); 2.5022 (8.9); 2.4977 (4.0); 2.2549 (1.1); 1.2338 (1.0); - 0.0002 (6.7) |
| 1-011: $^{1}$H-NMR(400.0 MHz, $d_6$-DMSO):<br>$\delta$= 13.8885 (0.8); 8.7393 (11.2); 8.7384 (11.3); 8.6121 (16.0); 8.0773 (12.0); 8.0763 (12.0); 5.7582 (0.7); 2.5161 (4.7); 2.5115 (10.6); 2.5069 (15.0); 2.5024 (10.6); 2.4978 (4.8); -0.0002 (9.7) |
| 1-013: $^{1}$H-NMR(400.0 MHz, CDCl3):<br>$\delta$= 11.9483 (1.5); 8.9272 (13.3); 7.8381 (8.8); 7.6760 (16.0); 7.5197 (0.5); 7.2945 (0.6); 7.2606 (104.6); 6.9966 (0.6); 1.5723 (14.6); 0.1462 (0.6); 0.0079 (4.1); -0.0002 (139.1); -0.0085 (4.2) |
| 1-014: $^{1}$H-NMR(400.0 MHz, CDCl3):<br>$\delta$= 9.3522 (0.5); 8.5180 (0.8); 8.5052 (16.0); 8.3353 (0.6); 7.8080 (3.8); 7.5195 (0.6); 7.2605 (84.0); 5.3001 (3.5); 3.5337 (0.6); 3.5250 (1.9); 3.4170 (0.8); 3.2045 (0.9); 1.5660 (3.5); 1.3284 (0.6); 1.2843 (1.0); 1.2545 (6.3); 0.8787 (0.7); 0.0079 (3.7); -0.0002 (110.3); -0.0085 (2.9) |
| 1-015: $^{1}$H-NMR(400.0 MHz, CDCl3):<br>$\delta$= 8.8373 (16.0); 7.7229 (2.9); 7.2603 (49.1); 3.5292 (0.6); 1.5578 (3.0); 0.0080 (1.9); -0.0002 (64.9); -0.0085 (2.0) |
| I-016: $^{1}$H-NMR(400.6 MHz, $d_6$-DMSO):<br>$\delta$= 13.9095 (0.6); 8.7216 (5.4); 8.7208 (5.6); 8.7180 (5.7); 8.7171 (5.2); 8.6130 (16.0); 8.0509 (5.4); 8.0501 (4.8); 8.0387 (5.7); 3.3261 (10.7); 2.5280 (0.6); 2.5247 (1.3); 2.5201 (1.8); 2.5114 (23.0); 2.5068 (49.9); 2.5022 (69.6); 2.4976 (47.2); 2.4930 (20.1); 1.9891 (1.2); 1.1749 (0.7); 0.8580 (0.7); 0.0079 (1.5); 0.0046 (0.6); -0.0002 (54.2); -0.0028 (1.5); -0.0035 (0.9); -0.0085 (1.4) |
| 1-017: $^{1}$H-NMR(400.6 MHz, $d_6$-DMSO): |

(continued)

| |
|---|
| δ= 13.9251 (0.5); 8.6141 (16.0); 8.5066 (9.6); 8.4945 (10.2); 7.8968 (4.4); 7.8848 (8.0); 7.8729 (4.3); 3.4280 (0.8); 3.3274 (8.9); 2.5254 (1.2); 2.5208 (1.6); 2.5121 (18.3); 2.5075 (39.4); 2.5029 (55.1); 2.4983 (37.2); 2.4937 (15.8); 1.9895 (0.5); 0.0080 (1.2); 0.0040 (0.6); 0.0023 (1.6); -0.0002 (42.0); -0.0025 (1.4); -0.0085 (1.0) |
| 1-018: $^{1}$H-NMR(400.6 MHz, $d_6$-DMSO):<br>δ= 13.9385 (0.9); 8.6132 (16.0); 8.4222 (3.8); 8.4196 (3.9); 8.4098 (4.2); 8.4072 (4.0); 7.8119 (4.8); 7.8105 (4.8); 7.7995 (4.7); 7.7981 (4.7); 4.0382 (0.9); 4.0205 (0.9); 3.3258 (5.1); 2.5248 (1.4); 2.5201 (1.7); 2.5114 (21.4); 2.5068 (47.1); 2.5022 (66.3); 2.4976 (45.2); 2.4930 (19.5); 1.9892 (4.5); 1.2586 (0.6); 1.2470 (1.5); 1.1927 (1.4); 1.1750 (2.8); 1.1572 (1.4); 0.8750 (0.8); 0.8582 (3.5); 0.8405 (1.1); 0.0271 (0.7); 0.0101 (0.6); 0.0079 (3.5); 0.0062 (1.1); 0.0053 (1.3); 0.0045 (1.6); 0.0036 (2.1); -0.0002 (123.2); -0.0029 (3.0); -0.0036 (1.7); -0.0045 (0.7); -0.0085 (3.0) |
| 1-019: $^{1}$H-NMR(400.6 MHz, $d_6$-DMSO):<br>δ= 13.8862 (0.9); 8.6060 (12.3); 8.6049 (16.0); 8.5941 (10.1); 7.8594 (9.8); 7.8474 (9.6); 3.3498 (0.8); 3.3215 (88.1); 2.6749 (0.8); 2.6702 (1.1); 2.6656 (0.8); 2.5407 (1.0); 2.5341 (1.1); 2.5295 (1.1); 2.5241 (2.7); 2.5194 (3.4); 2.5106 (57.4); 2.5060 (127.7); 2.5014 (180.3); 2.4968 (122.9); 2.4922 (53.8); 2.4802 (1.1); 2.3331 (0.7); 2.3284 (1.1); 2.3238 (0.7); 0.1458 (0.7); 0.0277 (1.8); 0.0104 (0.5); 0.0096 (0.7); 0.0080 (7.3); 0.0065 (1.4); 0.0057 (1.4); 0.0049 (1.8); 0.0041 (2.4); 0.0032 (4.1); 0.0024 (8.0); 0.0016 (12.4); -0.0002 (262.1); -0.0026 (10.0); -0.0033 (7.0); -0.0041 (3.9); -0.0050 (2.8); -0.0058 (2.3); -0.0066 (1.9); -0.0075 (2.2); -0.0085 (6.8); -0.0106 (1.2); -0.0114 (1.0); -0.0122 (0.8); -0.0130 (0.7); -0.0138 (0.6); -0.0146 (0.6); -0.0154 (0.6); -0.0162 (0.6); -0.0169 (0.5); -0.0177 (0.5); -0.0225 (0.5); -0.0257 (0.6); -0.1492 (0.7) |
| 1-020: $^{1}$H-NMR(400.6 MHz, $d_6$-DMSO):<br>δ= 13.8260 (0.9); 8.5997 (16.0); 8.5899 (9.3); 8.5781 (9.8); 7.7978 (9.5); 7.7860 (9.3); 3.3543 (0.5); 3.3261 (94.0); 2.6707 (0.5); 2.5246 (1.4); 2.5199 (1.8); 2.5111 (27.0); 2.5066 (59.9); 2.5019 (84.2); 2.4973 (57.4); 2.4928 (25.1); 0.0079 (2.2); -0.0002 (90.1); -0.0027 (3.0); -0.0052 (0.8); -0.0060 (0.6); -0.0069 (0.6); -0.0085 (2.5) |
| I-026: $^{1}$H-NMR(400.6 MHz, CDCl3):<br>δ= 8.6297 (1.4); 8.6173 (1.4); 8.4802 (2.1); 7.8709 (2.9); 7.5055 (1.3); 7.5042 (1.2); 7.4931 (1.3); 7.4918 (1.2); 7.2644 (5.6); 2.2443 (0.6); 1.3265 (16.0); 1.2954 (16.0); 1.2542 (0.7); 1.2490 (0.6); 1.1127 (1.1); 1.1085 (1.2); 1.1036 (0.6); 1.0967 (0.7); 1.0913 (1.2); 1.0873 (1.2); 1.0756 (0.6); 0.8799 (0.6); 0.8681 (1.4); 0.8647 (1.2); 0.8549 (1.2); 0.8516 (1.4); -0.0002 (9.0) |
| 1-027: $^{1}$H-NMR(400.6 MHz, $d_6$-DMSO):<br>δ= 13.8870 (0.8); 8.6435 (6.1); 8.6107 (16.0); 7.7685 (4.7); 7.7626 (4.6); 7.7618 (4.5); 3.3209 (12.1); 2.6747 (0.7); 2.6700 (1.0); 2.6654 (0.7); 2.5238 (2.8); 2.5192 (3.8); 2.5104 (52.6); 2.5058 (116.6); 2.5012 (164.6); 2.4966 (115.0); 2.4920 (51.5); 2.3330 (0.7); 2.3283 (1.0); 2.3236 (0.7); 0.1457 (0.5); 0.0104 (0.5); 0.0080 (4.9); 0.0056 (1.1); 0.0048 (1.4); -0.0002 (183.6); -0.0085 (5.1); -0.1494 (0.5) |
| 1-028: $^{1}$H-NMR(400.6 MHz, $d_6$-DMSO):<br>δ= 8.6329 (3.3); 8.6074 (1.8); 8.5958 (1.9); 8.5757 (6.8); 7.6351 (2.0); 7.6227 (2.0); 5.7569 (0.7); 3.3203 (16.0); 2.6701 (0.7); 2.5238 (1.5); 2.5192 (2.2); 2.5104 (36.8); 2.5058 (81.1); 2.5013 (113.5); 2.4967 (80.0); 2.4922 (36.6); 2.4001 (11.3); 2.3283 (0.7); 0.0079 (2.8); -0.0002 (95.2); -0.0085 (3.0) |
| 1-029: $^{1}$H-NMR(400.6 MHz, $d_6$-DMSO):<br>δ= 8.8393 (4.0); 8.8348 (4.0); 8.6683 (2.7); 8.6656 (2.5); 8.6562 (2.8); 8.6535 (2.6); 8.6017 (11.9); 7.8628 (2.1); 7.8618 (1.9); 7.8505 (2.2); 7.8494 (2.2); 7.8478 (2.6); 7.8356 (2.1); 3.3229 (16.0); 2.6708 (0.5); 2.5246 (1.5); 2.5199 (2.1); 2.5111 (29.4); 2.5066 (65.1); 2.5020 (91.6); 2.4974 (64.2); 2.4928 (28.9); 2.3291 (0.6); 0.0080 (2.7); -0.0002 (97.6); -0.0085 (2.8) |
| 1-030: $^{1}$H-NMR(400.6 MHz, $d_6$-DMSO):<br>δ= 13.9352 (0.6); 8.6196 (16.0); 8.5306 (2.7); 8.5274 (5.1); 8.5240 (3.1); 7.7554 (2.4); 7.7478 (2.7); 7.7448 (3.0); 7.7371 (2.6); 3.3336 (0.6); 3.1697 (0.6); 2.5260 (0.7); 2.5213 (0.9); 2.5125 (12.8); 2.5080 (28.6); 2.5034 (40.6); 2.4988 (28.5); 2.4942 (12.8); 0.0079 (1.4); 0.0046 (0.5); 0.0038 (0.7); 0.0029 (1.2); 0.0021 (2.0); -0.0002 (49.4); -0.0028 (2.3); -0.0036 (1.6); -0.0044 (1.0); - 0.0052 (0.8); -0.0060 (0.6); -0.0068 (0.6); -0.0085 (1.5) |
| I-031: $^{1}$H-NMR(400.6 MHz, $d_6$-DMSO): |

(continued)

| |
|---|
| δ= 13.9586 (0.6); 8.6181 (16.0); 8.2662 (3.0); 8.2629 (2.9); 8.2536 (3.2); 8.2503 (3.0); 7.8169 (4.3); 7.8063 (4.8); 7.8044 (4.6); 7.7938 (4.0); 3.3257 (5.1); 2.6750 (0.6); 2.6704 (0.8); 2.6658 (0.6); 2.5242 (2.2); 2.5196 (3.0); 2.5108 (43.5); 2.5062 (97.1); 2.5016 (137.3); 2.4970 (95.5); 2.4925 (43.0); 2.4700 (0.6); 2.4654 (0.7); 2.3333 (0.6); 2.3287 (0.8); 2.3240 (0.6); 0.0079 (3.8); 0.0063 (0.8); 0.0054 (0.9); 0.0045 (1.2); -0.0002 (142.3); -0.0052 (2.0); -0.0060 (1.5); -0.0069 (1.3); -0.0085 (4.1); -0.0108 (0.6); -0.0116 (0.5) |
| 1-032: $^1$H-NMR(400.6 MHz, $d_6$-DMSO):<br>δ= 8.6353 (16.0); 7.7269 (2.1); 7.7204 (3.9); 7.7140 (2.0); 3.3335 (1.9); 3.1688 (0.5); 2.5249 (1.0); 2.5203 (1.3); 2.5115 (17.9); 2.5069 (39.8); 2.5024 (56.2); 2.4978 (39.2); 2.4932 (17.5); 1.2345 (0.7); 0.0080 (1.6); -0.0002 (54.4); -0.0058 (0.6); -0.0085 (1.5) |
| 1-033: $^1$H-NMR(400.6 MHz, $d_6$-DMSO):<br>δ= 8.5932 (5.4); 8.3884 (1.8); 8.3852 (1.8); 8.3842 (1.8); 7.2876 (1.7); 7.2866 (1.6); 7.2762 (1.9); 7.2752 (1.6); 3.8980 (14.0); 3.3203 (16.0); 2.6701 (0.5); 2.5238 (1.2); 2.5192 (1.6); 2.5104 (27.2); 2.5059 (61.0); 2.5013 (86.3); 2.4967 (60.2); 2.4921 (27.2); 2.3283 (0.5); 0.0080 (2.4); 0.0046 (0.7); -0.0002 (91.0); -0.0051 (1.4); -0.0060 (1.2); -0.0068 (1.0); -0.0085 (2.7) |
| 1-034: $^1$H-NMR(400.6 MHz, CDCl3):<br>δ= 11.8380 (0.6); 8.1731 (10.3); 7.9901 (16.0); 7.2611 (69.0); 7.2563 (10.0); 7.2488 (8.6); 2.2049 (0.6); 2.1918 (1.3); 2.1841 (1.5); 2.1708 (2.7); 2.1572 (1.5); 2.1498 (1.5); 2.1365 (0.8); 1.6397 (0.9); 1.6042 (1.5); 1.1672 (2.9); 1.1549 (6.4); 1.1513 (7.4); 1.1462 (3.8); 1.1395 (3.9); 1.1339 (6.9); 1.1303 (6.8); 1.1185 (3.3); 0.8829 (3.9); 0.8711 (8.0); 0.8679 (7.6); 0.8578 (7.3); 0.8547 (8.4); 0.8420 (2.7); 0.0080 (3.0); -0.0002 (102.8); -0.0084 (3.8) |
| 1-035: $^1$H-NMR(400.6 MHz, CDCl3):<br>δ= 12.0609 (1.1); 8.2955 (12.1); 7.9635 (16.0); 7.5908 (13.7); 7.5895 (13.7); 7.2611 (62.9); 2.2315 (0.6); 2.2190 (1.3); 2.2111 (1.4); 2.2059 (0.9); 2.1980 (2.7); 2.1902 (0.9); 2.1846 (1.5); 2.1770 (1.5); 2.1635 (0.7); 1.6394 (1.6); 1.6039 (1.7); 1.5726 (6.1); 1.5252 (0.5); 1.4281 (0.7); 1.3844 (0.8); 1.1804 (2.3); 1.1680 (5.1); 1.1643 (6.0); 1.1594 (3.1); 1.1523 (3.2); 1.1468 (5.6); 1.1432 (5.5); 1.1313 (2.8); 0.8972 (2.9); 0.8851 (6.5); 0.8819 (6.3); 0.8720 (5.6); 0.8687 (7.0); 0.8559 (2.2); 0.0080 (2.7); -0.0002 (93.4); -0.0085 (2.8) |
| I-036: $^1$H-NMR(400.6 MHz, CDCl3):<br>δ= 11.7896 (0.6); 8.7882 (14.2); 8.6652 (16.0); 7.8906 (9.5); 7.5189 (1.0); 7.2605 (189.0); 6.9969 (1.1); 2.4209 (0.7); 1.5554 (13.4); 1.2552 (4.6); 0.8799 (0.7); 0.1457 (1.0); 0.0389 (0.9); 0.0333 (1.1); 0.0285 (1.2); 0.0199 (1.0); 0.0080 (8.5); 0.0056 (3.5); -0.0002 (268.5); -0.0067 (4.0); -0.0084 (8.6); -0.0115 (1.7); -0.0178 (0.6); -0.1494 (0.9) |
| 1-037: $^1$H-NMR(400.6 MHz, $d_6$-DMSO):<br>δ= 13.6950 (0.8); 8.7741 (8.3); 8.7635 (1.1); 8.5890 (5.2); 8.5803 (10.4); 8.5156 (1.0); 7.9407 (0.9); 7.8458 (2.0); 7.7003 (4.7); 7.6880 (4.5); 5.7575 (1.0); 3.3269 (12.8); 3.2503 (1.4); 3.1063 (2.2); 3.0880 (6.7); 3.0697 (6.8); 3.0515 (2.2); 2.5073 (36.5); 2.5034 (45.8); 1.2998 (0.6); 1.2590 (1.0); 1.2313 (8.8); 1.2129 (16.0); 1.1947 (7.5); 1.0482 (0.6); 1.0325 (0.7); 0.8547 (0.8); 0.8366 (0.7); 0.8285 (0.5); 0.0078 (0.9); 0.0016 (13.6); -0.0002 (17.8); -0.0085 (0.6) |
| 1-038: $^1$H-NMR(400.6 MHz, $d_6$-DMSO):<br>δ= 13.8707 (0.8); 8.7166 (14.7); 8.6074 (15.6); 8.5160 (0.6); 8.1841 (16.0); 3.3277 (9.5); 2.5256 (1.0); 2.5210 (1.4); 2.5122 (19.9); 2.5078 (43.5); 2.5034 (60.7); 2.4990 (44.9); 2.4948 (21.6); 1.2348 (0.7); 0.0079 (0.8); -0.0002 (25.6); -0.0033 (3.7); - 0.0085 (0.8) |
| 1-039: $^1$H-NMR(400.6 MHz, $d_6$-DMSO):<br>δ= 13.8682 (1.5); 9.1436 (9.9); 9.1258 (5.9); 9.1134 (6.1); 8.5907 (11.0); 7.9670 (5.3); 7.9547 (5.2); 3.5664 (2.0); 3.5479 (6.8); 3.5294 (6.9); 3.5110 (2.2); 3.4231 (2.4); 3.3252 (12.5); 3.1688 (0.7); 2.5243 (0.8); 2.5196 (1.1); 2.5109 (17.2); 2.5065 (38.1); 2.5021 (53.6); 2.4977 (40.1); 2.4934 (19.5); 1.9891 (0.7); 1.2355 (0.8); 1.2226 (7.4); 1.2041 (16.0); 1.1857 (7.2); 1.1756 (0.8); 0.0080 (0.7); -0.0002 (21.9); -0.0035 (3.2); -0.0072 (0.7) |
| 1-040: $^1$H-NMR(400.6 MHz, $d_6$-DMSO):<br>δ= 13.8949 (0.6); 9.3273 (15.0); 8.6127 (15.7); 8.2688 (16.0); 3.3398 (1.8); 3.1691 (2.1); 2.5251 (0.9); 2.5203 (1.3); 2.5116 (18.0); 2.5073 (39.5); 2.5028 (55.2); 2.4985 (41.2); 2.4943 (20.1); 1.2355 (1.4); 0.0080 (0.7); -0.0002 (22.7); -0.0035 (3.4); - 0.0071 (0.7) |
| I-041: $^1$H-NMR(400.6 MHz, $d_6$-DMSO): |

(continued)

| |
|---|
| δ= 8.5800 (2.8); 8.4913 (2.9); 7.2848 (2.9); 3.9064 (8.3); 3.8992 (1.2); 3.3240 (16.0); 2.5067 (39.2); 2.5029 (49.6); 1.2586 (0.5); 1.2361 (1.4); 0.0079 (0.9); 0.0017 (14.9); -0.0002 (17.5); -0.0062 (0.6); -0.0085 (0.6) |
| 1-042: $^{1}$H-NMR(400.6 MHz, CDCl3):<br>δ= 9.4654 (1.6); 9.0800 (0.9); 9.0673 (0.9); 8.8321 (2.5); 8.3168 (1.5); 7.6621 (1.2); 7.6498 (0.9); 7.5211 (0.6); 7.4042 (2.7); 7.2608 (115.0); 7.1110 (3.0); 6.9972 (0.7); 4.0007 (16.0); 1.6676 (0.7); 0.1463 (0.8); 0.0079 (6.7); 0.0010 (137.9); -0.0002 (170.9); -0.1486 (0.8) |

The present invention furthermore provides the use of one or more compounds of the general formula (I), salts or N-oxides thereof, as defined above, preferably in one of the embodiments identified as preferred or particularly preferred, in particular one or more compounds of the formulae (1-001) to (1-042), salts or N-oxides thereof, in each case as defined above, as herbicide and/or plant growth regulator, preferably in crops of useful plants and/or ornamental plants.

The present invention furthermore provides a method for controlling harmful plants and/or for regulating the growth of plants, characterized in that an effective amount

- of one or more compounds of the general formula (I), salts or N-oxides thereof, as defined above, preferably in one of the embodiments identified as preferred or particularly preferred, in particular one or more compounds of the formulae (1-001) to (1-042), salts or N-oxides thereof, in each case as defined above, or

- of a composition according to the invention, as defined below,

is applied to the (harmful) plants, seeds of (harmful) plants, the soil in which or on which the (harmful) plants grow or the area under cultivation.

The present invention also provides a method for controlling unwanted plants, preferably in crops of useful plants, characterized in that an effective amount

- of one or more compounds of the general formula (I), salts or N-oxides thereof, as defined above, preferably in one of the embodiments identified as preferred or particularly preferred, in particular one or more compounds of the formulae (1-001) to (1-042), salts or N-oxides thereof, in each case as defined above, or

- of a composition according to the invention, as defined below,

is applied to unwanted plants (for example harmful plants such as mono- or dicotyledonous weeds or unwanted crop plants), the seed of the unwanted plants (i.e. plant seeds, for example grains, seeds or vegetative propagation organs such as tubers or shoot parts with buds), the soil in which or on which the unwanted plants grow (for example the soil of crop land or non-crop land) or the area under cultivation (i.e. the area on which the unwanted plants will grow).

The present invention furthermore also provides methods for regulating the growth of plants, preferably of useful plants, characterized in that an effective amount

- of one or more compounds of the general formula (I), salts or N-oxides thereof, as defined above, preferably in one of the embodiments identified as preferred or particularly preferred, in particular one or more compounds of the formulae (1-001) to (1-042), salts or N-oxides thereof, in each case as defined above, or

- of a composition according to the invention, as defined below,

is applied to the plant, the seed of the plant (i.e. plant seed, for example grains, seeds or vegetative propagation organs such as tubers or shoot parts with buds), the soil in which or on which the plants grow (for example the soil of crop land or non-crop land) or the area under cultivation (i.e. the area on which the plants will grow).

In this context, the compounds according to the invention or the compositions according to the invention can be applied for example by pre-sowing (if appropriate also by incorporation into the soil), pre-emergence and/or post-emergence processes. Specific examples of some representatives of the monocotyledonous and dicotyledonous weed flora which can be controlled by the compounds according to the invention are as follows, though there is no intention to restrict the enumeration to particular species.

In a method according to the invention for controlling harmful plants or for regulating the growth of plants, one or more compounds of the general formula (I), salts or N-oxides thereof are preferably employed for controlling harmful

plants or for regulating growth in crops of useful plants or ornamental plants, where in a preferred embodiment the useful plants or ornamental plants are transgenic plants.

The compounds of the general formula (I) according to the invention and/or their salts and N-oxides are suitable for controlling the following genera of monocotyledonous and dicotyledonous harmful plants:

Monocotyledonous harmful plants of the genera: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dicotyledonous harmful plants of the genera: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

When the compounds according to the invention are applied to the soil surface before germination of the harmful plants (weed grasses and/or broad-leaved weeds) (pre-emergence method), either the seedlings of the weed grasses or broad-leaved weeds are prevented completely from emerging or they grow until they have reached the cotyledon stage, but then stop growing and eventually, after three to four weeks have elapsed, die completely.

If the active compounds are applied post-emergence to the green parts of the plants, growth stops after the treatment, and the harmful plants remain at the growth stage at the time of application, or they die completely after a certain time, so that in this manner competition by the weeds, which is harmful to the crop plants, is eliminated very early and in a sustained manner.

Although the compounds according to the invention display an outstanding herbicidal activity against monocotyledonous and dicotyledonous weeds, crop plants of economically important crops, for example dicotyledonous crops of the genera Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, or monocotyledonous crops of the genera Allium, Ananas, Asparagus, Avena, Hordeum, Oryza, Panicum, Saccharum, Secale, Sorghum, triticale, triticum, Zea, are damaged only to an insignificant extent, or not at all, depending on the structure of the respective compound according to the invention and its application rate. For these reasons, the present compounds are very suitable for selective control of unwanted plant growth in plant crops such as agriculturally useful plants or ornamental plants.

In addition, the compounds of the invention (depending on their particular structure and the application rate deployed) have outstanding growth-regulating properties in crop plants. They intervene in the plants' own metabolism with regulatory effect and can thus be used for the controlled influencing of plant constituents and to facilitate harvesting, for example by triggering desiccation and stunted growth. Furthermore, they are also suitable for the general control and inhibition of unwanted vegetative growth without killing the plants in the process. Inhibition of vegetative growth plays a major role for many mono- and dicotyledonous crops since, for example, this can reduce or completely prevent lodging.

By virtue of their herbicidal and plant growth regulatory properties, the active compounds can also be used to control harmful plants in crops of genetically modified plants or plants modified by conventional mutagenesis. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material.

It is preferred with a view to transgenic crops to use the compounds according to the invention and/or their salts and N-oxides in economically important transgenic crops of useful plants and ornamentals, for example of cereals such as wheat, barley, rye, oats, millet, rice and corn or else crops of sugar beet, cotton, soybean, oilseed rape, potato, tomato, peas and other vegetables.

It is preferred to employ the compounds according to the invention as herbicides in crops of useful plants which are resistant, or have been made resistant by recombinant means, to the phytotoxic effects of the herbicides.

By virtue of their herbicidal and plant growth regulatory properties, the active compounds can also be used to control harmful plants in crops of genetically modified plants which are known or are yet to be developed. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid

composition in the harvested material. Further special properties may be tolerance or resistance to abiotic stressors, for example heat, cold, drought, salinity and ultraviolet radiation.

Preference is given to the use of the compounds of the general formula (I) according to the invention and/or their salts and N-oxides in economically important transgenic crops of useful plants and ornamental plants, for example of cereals such as wheat, barley, rye, oats, triticale, millet, rice, cassava and corn, or else crops of sugar beet, cotton, soybean, oilseed rape, potatoes, tomatoes, peas and other vegetables.

The compounds of the general formula (I) can preferably be used as herbicides in crops of useful plants which are resistant, or have been made resistant by recombinant means, to the phytotoxic effects of the herbicides.

Conventional ways of producing novel plants which have modified properties in comparison to existing plants consist, for example, in traditional cultivation methods and the generation of mutants. Alternatively, novel plants with altered properties can be generated with the aid of recombinant methods.

A large number of molecular-biological techniques by means of which novel transgenic plants with modified properties can be generated are known to the person skilled in the art. For such recombinant manipulations, nucleic acid molecules which allow mutagenesis or sequence alteration by recombination of DNA sequences can be introduced into plasmids. With the aid of standard methods, it is possible, for example, to undertake base exchanges, remove parts of sequences or add natural or synthetic sequences. To connect the DNA fragments to each other, adapters or linkers may be added to the fragments.

For example, the generation of plant cells with a reduced activity of a gene product can be achieved by expressing at least one corresponding antisense RNA, a sense RNA for achieving a cosuppression effect, or by expressing at least one suitably constructed ribozyme which specifically cleaves transcripts of the abovementioned gene product.

To this end, it is firstly possible to use DNA molecules which encompass the entire coding sequence of a gene product inclusive of any flanking sequences which may be present, and also DNA molecules which only encompass portions of the coding sequence, in which case it is necessary for these portions to be long enough to have an antisense effect in the cells. It is also possible to use DNA sequences which have a high degree of homology to the coding sequences of a gene product, but are not completely identical to them.

When expressing nucleic acid molecules in plants, the protein synthesized may be localized in any desired compartment of the plant cell. However, to achieve localization in a particular compartment, it is possible, for example, to join the coding region to DNA sequences which ensure localization in a particular compartment. Such sequences are known to those skilled in the art (see, for example, Braun et al., EMBO J. 11 (1992), 3219-3227). The nucleic acid molecules can also be expressed in the organelles of the plant cells.

The transgenic plant cells can be regenerated by known techniques to give rise to entire plants. In principle, the transgenic plants may be plants of any desired plant species, i.e. not only monocotyledonous but also dicotyledonous plants.

Thus, transgenic plants can be obtained whose properties are altered by overexpression, suppression or inhibition of homologous (= natural) genes or gene sequences or expression of heterologous (= foreign) genes or gene sequences.

It is preferred to employ the compounds (I) according to the invention in transgenic crops which are resistant to growth regulators such as, for example, dicamba, or to herbicides which inhibit essential plant enzymes, for example acetolactate synthases (ALS), EPSP synthases, glutamine synthases (GS) or hydroxyphenylpyruvate dioxygenases (HPPD), or to herbicides from the group of the sulfonylureas, glyphosate, glufosinate or benzoylisoxazoles and analogous active compounds.

When the active compounds of the invention are employed in transgenic crops, not only do the effects toward harmful plants observed in other crops occur, but frequently also effects which are specific to application in the particular transgenic crop, for example an altered or specifically widened spectrum of weeds which can be controlled, altered application rates which can be used for the application, preferably good combinability with the herbicides to which the transgenic crop is resistant, and influencing of growth and yield of the transgenic crop plants.

The invention therefore also relates to the use of the compounds of the general formula (I) according to the invention and/or their salts and N-oxides as herbicides for controlling harmful plants in crops of useful plants or ornamentals, optionally in transgenic crop plants.

Preference is given to the use in cereals, here preferably corn, wheat, barley, rye, oats, millet or rice, by the pre- or post-emergence method.

Preference is also given to the use in soybeans by the pre- or post-emergence method.

The use according to the invention for the control of harmful plants or for growth regulation of plants also includes the case in which the active compound of the general formula (I) or its salt is not formed from a precursor substance ("prodrug") until after application on the plant, in the plant or in the soil. The invention also provides for the use of one or more compounds of the general formula (I), salts or N-oxides thereof or of a composition according to the invention (as defined below) (in a method) for controlling harmful plants or for regulating the growth of plants which comprises applying an effective amount of one or more compounds of the general formula (I), salts or N-oxides thereof

onto the plants (harmful plants, if appropriate together with the useful plants), plant seeds, the soil in which or on which the plants grow or the area under cultivation.

The invention also provides a herbicidal and/or plant growth-regulating composition, characterized in that the composition comprises

(a) one or more compounds of the general formula (I), salts or N-oxides thereof, as defined above, preferably in one of the embodiments identified as preferred or particularly preferred, in particular one or more compounds of the formulae (1-001) to (1-042), salts or N-oxides thereof, in each case as defined above,and

(b) one or more further substances selected from groups (i) and/or (ii):

(i) one or more further agrochemically active substances, preferably selected from the group consisting of insecticides, acaricides, nematicides, further herbicides (i.e. those not corresponding to the general formula (I) defined above), fungicides, safeners, fertilizers and/or further growth regulators,

(ii) one or more formulation auxiliaries customary in crop protection.

Here, the further agrochemically active substances of component (i) of a composition according to the invention are preferably selected from the group of substances mentioned in "The Pesticide Manual", 16th edition, The British Crop Protection Council and the Royal Soc. of Chemistry, 2012.

A herbicidal or plant growth-regulating composition according to the invention comprises preferably one, two, three or more formulation auxiliaries (ii) customary in crop protection selected from the group consisting of surfactants, emulsifiers, dispersants, film-formers, thickeners, inorganic salts, dusting agents, carriers solid at 25 °C and 1013 mbar, preferably adsorbent granulated inert materials, wetting agents, antioxidants, stabilizers, buffer substances, antifoam agents, water, organic solvents, preferably organic solvents miscible with water in any ratio at 25 °C and 1013 mbar.

The compounds of general formula (I) according to the invention can be used in the form of wettable powders, emulsifiable concentrates, sprayable solutions, dusting products or granules in the customary formulations. The invention therefore also provides herbicidal and plant growth-regulating compositions which comprise compounds of the general formula (I), salts or N-oxides thereof.

The compounds of the general formula (I), salts or N-oxides thereof can be formulated in various ways according to which biological and/or physicochemical parameters are required. Possible formulations include, for example: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes.

These individual formulation types and the formulation assistants, such as inert materials, surfactants, solvents and further additives, are known to the person skilled in the art and are described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd ed., J. Wiley & Sons, N.Y.; C. Marsden, "Solvents Guide", 2nd ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflachenaktive Äthylenoxidaddukte" [Interface-active Ethylene Oxide Adducts], Wiss. Verlagsgesellschaft, Stuttgart 1976; Winnacker-Küchler, "Chemische Technologie" [Chemical Technology], volume 7, C. Hanser Verlag Munich, 4th Ed. 1986.

Wettable powders are preparations which can be dispersed uniformly in water and, in addition to the active compound, apart from a diluent or inert substance, also comprise surfactants of the ionic and/or nonionic type (wetting agents, dispersants), for example polyoxyethylated alkylphenols, polyoxyethylated fatty alcohols, polyoxyethylated fatty amines, fatty alcohol polyglycol ether sulfates, alkanesulfonates, alkylbenzenesulfonates, sodium lignosulfonate, sodium 2,2'-dinaphthylmethane-6,6'-disulfonate, sodium dibutylnaphthalenesulfonate or else sodium oleoylmethyltaurate. To produce the wettable powders, the herbicidally active compounds are finely ground, for example in customary apparatuses such as hammer mills, blower mills and air-jet mills, and simultaneously or subsequently mixed with the formulation auxiliaries.

Emulsifiable concentrates are produced by dissolving the active compound in an organic solvent, for example butanol, cyclohexanone, dimethylformamide, xylene, or else relatively high-boiling aromatics or hydrocarbons or mixtures of the organic solvents, with addition of one or more ionic and/or nonionic surfactants (emulsifiers). Examples of emulsifiers which may be used are: calcium alkylarylsulfonates such as calcium dodecylbenzenesulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters, or polyoxyethylene

sorbitan esters, for example polyoxyethylene sorbitan fatty acid esters.

Dusting products are obtained by grinding the active compound with finely distributed solids, for example talc, natural clays, such as kaolin, bentonite and pyrophyllite, or diatomaceous earth.

Suspension concentrates may be water- or oil-based. They may be prepared, for example, by wetgrinding by means of commercial bead mills and optional addition of surfactants as have, for example, already been listed above for the other formulation types.

Emulsions, for example oil-in-water emulsions (EW), can be produced, for example, by means of stirrers, colloid mills and/or static mixers using aqueous organic solvents and optionally surfactants as already listed above, for example, for the other formulation types.

Granules can be produced either by spraying the active compound onto adsorptive granular inert material or by applying active compound concentrates to the surface of carriers, such as sand, kaolinites or granular inert material, by means of adhesives, for example polyvinyl alcohol, sodium polyacrylate or else mineral oils. Suitable active compounds can also be granulated in the manner customary for the production of fertilizer granules - if desired as a mixture with fertilizers.

Water-dispersible granules are produced generally by the customary processes such as spray-drying, fluidized-bed granulation, pan granulation, mixing with high-speed mixers and extrusion without solid inert material.

For the production of pan, fluidized-bed, extruder and spray granules, see e.g. processes in "Spray Drying Handbook" 3rd Ed. 1979, G. Goodwin Ltd., London, J.E. Browning, "Agglomeration", Chemical and Engineering 1967, pages 147 ff; "Perry’s Chemical Engineer’s Handbook", 5th Ed., McGraw Hill, New York 1973, p. 8-57.

For further details regarding the formulation of crop protection compositions, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

The agrochemical preparations, preferably herbicidal or plant growth-regulating compositions, of the present invention preferably comprise a total amount of from 0.1 to 99% by weight, preferably 0.5 to 95% by weight, particularly preferably 1 to 90% by weight, especially preferably 2 to 80% by weight, of active compounds of the general formula (I), salts or N-oxides thereof.

In wettable powders, the active compound concentration is, for example, about 10 to 90% by weight, the remainder to 100% by weight consisting of customary formulation constituents. In emulsifiable concentrates, the active compound concentration may be about 1% to 90% and preferably 5% to 80% by weight. Formulations in the form of dusts comprise 1% to 30% by weight of active compound, preferably usually 5% to 20% by weight of active compound; sprayable solutions contain about 0.05% to 80% by weight, preferably 2% to 50% by weight of active compound. In the case of water-dispersible granules, the active compound content depends partially on whether the active compound is in liquid or solid form and on which granulation auxiliaries, fillers, etc., are used. In the water-dispersible granules, the content of active compound is, for example, between 1% and 95% by weight, preferably between 10% and 80% by weight.

In addition, the active compound formulations mentioned optionally comprise the respective customary stickers, wetters, dispersants, emulsifiers, penetrants, preservatives, antifreeze agents and solvents, fillers, carriers and dyes, defoamers, evaporation inhibitors and agents which influence the pH and the viscosity. Examples of formulation auxiliaries are described inter alia in "Chemistry and Technology of Agrochemical Formulations", ed. D.A. Knowles, Kluwer Academic Publishers (1998).

The compounds of the general formula (I), salts or N-oxides thereof can be used as such or in the form of their preparations (formulations) in a combination with other pesticidally active substances, for example insecticides, acaricides, nematicides, herbicides, fungicides, safeners, fertilizers and/or growth regulators, for example in the form of a finished formulation or of a tank mix. The combination formulations can be prepared on the basis of the abovementioned formulations, while taking account of the physical properties and stabilities of the active compounds to be combined.

Active compounds which can be employed in combination with the compounds of general formula (I) according to the invention in mixture formulations or in a tank mix are, for example, known active compounds based on inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoendesaturase, photosystem I, photosystem II, protoporphyrinogen oxidase, as described, for example, in Weed Research 26 (1986) 441-445 or "The Pesticide Manual", 16th edition, The British Crop Protection Council and the Royal Soc. of Chemistry, 2012 and literature cited therein.

Of particular interest is the selective control of harmful plants in crops of useful plants and ornamentals. Although the compounds of genarl formula (I) according to the invention have already demonstrated very good to adequate selectivity in a large number of crops, in principle, in some crops and in particular also in the case of mixtures with other, less selective herbicides, phytotoxicities on the crop plants may occur. In this connection, combinations of compounds of general formula (I) according to the invention are of particular interest which comprise the compounds of general formula (I) or their combinations with other herbicides or pesticides and safeners. The safeners, which are used in an antidotically effective amount, reduce the phytotoxic side effects of the herbicides/pesticides employed, for example in

economically important crops, such as cereals (wheat, barley, rye, corn, rice, millet), sugarbeet, sugarcane, oilseed rape, cotton and soybeans, preferably cereals.

The weight ratios of herbicide (mixture) to safener depend generally on the herbicide application rate and the efficacy of the safener in question and may vary within wide limits, for example in the range from 200:1 to 1:200, preferably 100:1 to 1:100, in particular 20:1 to 1:20. Analogously to the compounds (I) or mixtures thereof, the safeners can be formulated with further herbicides/pesticides and be provided and employed as a finished formulation or tank mix with the herbicides.

For application, the herbicide or herbicide/safener formulations present in commercial form are, if appropriate, diluted in a customary manner, for example in the case of wettable powders, emulsifiable concentrates, dispersions and water-dispersible granules with water. Dust-type preparations, granules for soil application or granules for scattering and sprayable solutions are not normally diluted further with other inert substances prior to application.

The application rate of the compounds of the general formula (I), salts or N-oxides thereof is affected to a certain extent by external conditions such as temperature, humidity, etc. Here, the application rate may vary within wide limits. For the application as a herbicide for controlling harmful plants, the total amount of compounds of the general formula (I), salts or N-oxides thereof are preferably in the range from 0.001 to 10.0 kg/ha, with preference in the range from 0.005 to 5 kg/ha, more preferably in the range from 0.01 to 1.5 kg/ha, in particular preferably in the range from 0.05 to 1 kg/ha. This applies both to the pre-emergence and the post-emergence application.

When the compounds of the general formula (I), salts or N-oxides thereof are used as plant growth regulators, for example as culm stabilizer for crop plants like those mentioned above, preferably cereal plants, such as wheat, barley, rye, triticale, millet, rice or corn, the total application rate is preferably in the range of from 0.001 to 2 kg/ha, preferably in the range of from 0.005 to 1 kg/ha, in particular in the range of from 10 to 500 g/ha, very particularly in the range from 20 to 250 g/ha. This applies both to the pre-emergence and the post-emergence application.

The application as culm stabilizer may take place at various stages of the growth of the plants. Preferred is, for example, the application after the tilling phase, at the beginning of the longitudinal growth.

As an alternative, application as plant growth regulator is also possible by treating the seed, which includes various techniques for dressing and coating seed. Here, the application rate depends on the particular techniques and can be determined in preliminary tests.

Active compounds which can be employed in combination with the compounds of the general formula (I) according to the invention in compositions according to the invention (for example in mixed formulations or in the tank mix) are, for example, known active compounds which are based on the inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoene desaturase, photosystem I, photosystem II, protoporphyrinogen oxidase, as are described in, for example, Weed Research 26 (1986) 441-445 or "The Pesticide Manual", 16th edition, The British Crop Protection Council and the Royal Soc. of Chemistry, 2012 and the literature cited therein. Known herbicides or plant growth regulators which can be combined with the compounds according to the invention are, for example, the following active compounds, where the compounds are designated either with the "common name" in accordance with the International Organization for Standardization (ISO) or with the chemical name or with the code number. They always encompass all of the application forms such as, for example, acids, salts, esters and also all isomeric forms such as stereoisomers and optical isomers, even if not explicitly mentioned.

Examples of such herbicidal mixing partners are:

Acetochlor, acifluorfen, acifluorfen-methyl, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, aminopyralid-dimethylammonium, aminopyralid-tripromine, amitrole, ammoniumsulfamate, anilofos, asulam, asulam-potassium, asulam sodium, atrazine, azafenidin, azimsulfuron, beflubutamid, (S)-(-)-beflubutamid, beflubutamid-M, benazolin, benazolin-ethyl, benazolin-dimethylammonium, benazolin-potassium, benfluralin, benfuresate, bensulfuron, bensulfuron-methyl, bensulide, bentazone, bentazone-sdium, benzobicyclon, benzofenap, bicyclopyrone, bifenox, bilanafos, bilanafos-sodium, bipyrazone, bispyribac, bispyribac-sodium, bixlozone, bromacil, bromacil-lithium, bromacil-sodium, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, cambendichlor, carbetamide, carfentrazone, carfentrazone-ethyl, chloramben, chloramben-ammonium, chloramben-diolamine, chlroamben-methyl, chloramben-methylammonium, chloramben-sodium, chlorbromuron, chlorfenac, chlorfenac-ammonium, chlorfenac-sodium, chlorfenprop, chlorfenprop-methyl, chlorflurenol, chlorflurenol-methyl, chloridazon, chlorimuron, chlorimuron-ethyl, chlorophthalim, chlorotoluron, chlorsulfuron, chlorthal, chlorthal-dimethyl, chlorthal-monomethyl, cinidon, cinidon-ethyl, cinmethylin, exo-(+)-cinmethylin, i.e. (1R,2S,4S)-4-isopropyl-1-methyl-2-[(2-methylbenzyl)oxy]-7-oxabicyclo[2.2.1]heptane, exo-(-)-cinmethylin, i.e. (1R,2S,4S)-4-isopropyl-1-methyl-2-[(2-methylbenzyl)oxy]-7-oxabicyclo[2.2.1]heptane, cinosulfuron, clacyfos, clethodim, clodinafop, clodinafop-ethyl, clodinafop-propargyl, clomazone, clomeprop, clopyralid, clopyralid-methyl, clopyralid-olamine, clopyralid-potassium, clopyralid-tripomine,

cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D (including theammonium, butotyl, -butyl, choline, diethylammonium, -dimethylammonium, -diolamine, -doboxyl, -dodecylammonium, etexyl, ethyl, 2-ethylhexyl, heptylammonium, isobutyl, isooctyl, isopropyl, isopropylammonium, lithium, meptyl, methyl, potassium, tetradecylammonium, triethylammonium, triisopropanolammonium, tripromine and trolamine salt thereof), 2,4-DB, 2,4-DB-butyl, -dimethylammonium, isooctyl, -potassium und -sodium, daimuron (dymron), dalapon, dalapon-calcium, dalapon-magnesium, dalapon-sodium, dazomet, dazomet-sodium, n-decanol, 7-deoxy-D-sedoheptulose, desmedipham, detosyl-pyrazolate (DTP), dicamba and its salts, e. g. dicamba-biproamine, dicamba-N,N-Bis(3-aminopropyl)methylamine, dicamba-butotyl, dicamba-choline, dicamba-diglycolamine, dicamba-dimethylammonium, dicamba-diethanolaminemmonium, dicamba-diethylammonium, dicamba-isopropylammonium, dicamba-methyl, dicamba-monoethanolaminedicamba-olamine, dicamba-potassium, dicamba-sodium, dicamba-triethanolamine, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-butotyl, dichlroprop-dimethylammonium, dichhlorprop-etexyl, dichlorprop-ethylammonium, dichlorprop-isoctyl, dichlorprop-methyl, dichlorprop-postassium, dichlorprop-sodium, dichlorprop-P, dichlorprop-P-dimethylammonium, dichlorprop-P-etexyl, dichlorprop-P-potassium, dichlorprop-sodium, diclofop, diclofop-methyl, diclofop-P, diclofop-P-methyl, diclosulam, difenzoquat, difenzoquat-metilsulfate, diflufenican, diflufenzopyr, diflufenzopyr-sodium, dimefuron, dimepiperate, dimesulfazet, dimethachlor, dimethametryn, dimethenamid, dimethenamid-P, dimetrasulfuron, dinitramine, dinoterb, dinoterb-acetate, diphenamid, diquat, diquat-dibromid, diquat-dichloride, dithiopyr, diuron, DNOC, DNOC-ammonium, DNOC-potassium, DNOC-sodium, endothal, endothal-diammonium, endothal-dipotassium, endothal-disodium, Epyrifenacil (S-3100), EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dione, fenoxaprop, fenoxaprop-P, fenoxaprop-ethyl, fenoxaprop-P-ethyl, fenoxasulfone, fenpyrazone, fenquinotrione, fentrazamide, flamprop, flamprop-isoproyl, flamprop-methyl, flamprop-M-isopropyl, flamprop-M-methyl, flazasulfuron, florasulam, florpyrauxifen, florpyrauxifen-benzyl, fluazifop, fluazifop-butyl, fluazifop-methyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium und -methyl, fluoroglycofen, fluoroglycofen-ethyl, flupropanate, flupropanate-sdium, flupyrsulfuron, flupyrsulfuron-methyl, flupyrsulfuron-methyl-sodium, fluridone, flurochloridone, fluroxypyr, fluroxypyr-butometyl, fluroxypyr-meptyl, flurtamone, fluthiacet, fluthiacet-methyl, fomesafen, fomesafen-sodium, foramsulfuron, foramsulfuron sodium salt, fosamine, fosamine-ammonium, glufosinate, glufosinate-ammonium, glufosinate-sodium, L-glufosinate-ammonium, L-glufosiante-sodium, glufosinate-P-sodium, glufosinate-P-ammonium, glyphosate, glyphosate-ammonium, -isopropylammonium, -diammonium, -dimethylammonium, -potassium, - sodium, sesquisodium and -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, haloxifop-sodium, hexazinone, HNPC-A8169, i.e. prop-2-yn-1-yl (2S)-2-{3-[(5-tert-butylpyridin-2-yl)oxy]phenoxy}propanoate, HW-02, i.e. 1-(Dimethoxyphosphoryl)-ethyl-(2,4-dichlorphenoxy)acetat, hydantocidin, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazaquin.methyl, imazethapyr, imazethapyr-immonium, imazosulfuron, indanofan, indaziflam, iodosulfuron, iodosulfuron-methyl, iodosulfuron-methyl-sodium, ioxynil, ioxynil-lithium, -octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, ketospiradox-potassium, lactofen, lancotrione, lenacil, linuron, MCPA, MCPA-butotyl, -butyl, -dimethylammonium, -diolamine, -2-ethylhexyl, -ethyl, -isobutyl, isoctyl, -isopropyl, -isopropylammonium, -methyl, olamine, -potassium, -sodium and -trolamine, MCPB, MCPB-methyl, -ethyl und -sodium, mecoprop, mecoprop-butotyl, mecoprop- demethylammonium, mecoprop-diolamine, mecoprop-etexyl, mecoprop-ethadyl, mecoprop-isoctyl, mecoprop-methyl, mecoprop-potassium, mecoprop-sodium, and mecoprop-trolamine, mecoprop-P, mecoprop-P-butotyl, -dimethylammonium, -2-ethylhexyl and -potassium, mefenacet, mefluidide, mefluidide-diolamine, mefluidide-potassium, mesosulfuron, mesosulfuron-methyl, mesosulfuron sodium salt, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinate, monolinuron, monosulfuron, monosulfuron-methyl, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, NC-656, i.e. 3-[(isopropylsulfonyl)methyl]-N-(5-methyl-1,3,4-oxadiazol-2-yl)-5-(trifluoromethyl)[1,2,4]triazolo[4,3-a]pyridine-8-carboxamide, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefone, oxyfluorfen, paraquat, paraquat-dichloride, paraquat-dimethylsulfate, pebulate, pendimethalin, penoxsulam, pentachlorphenol, pentoxazone, pethoxamid, petroleum oils, phenmedipham, phenmedipham-ethyl, picloram, picloram-dimethylammonium, picloram-etexyl, picloram-isoctyl, picloram-methyl, picloram-olamine, picloram-potassium, picloram-triethylammonium, picloram-tripromine, picloram-trolamine, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinclorac-dimethylammonium, quinclorac-methyl, quinmerac, quinoclamine, quizalofop, quizalofop-ethyl, quizalofop-P, quizalofop-P-ethyl, quizalofop-P-tefuryl, QYM201, i.e. 1- {2-chloro-3- [(3-cyclopropyl-5-hydroxy-1-methyl-1H-pyrazol-4-yl)carbonyl] -6-(trifluoromethyl)phenyl}piperidin-2-one, rimsulfuron, saflufenacil, sethoxydim, siduron, simazine, simetryn, SL-261, sulcotrione, sulfentrazone, sulfometuron, sulfometuron-methyl, sulfosulfuron, , SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (trichloro acetic acid) and its salts, e.g. TCA-ammonium, TCA-calcium, TCA-ethyl, TCA-magnesium, TCA-sodium, tebuthiuron, tefuryltrione, tembotrione, tepraloxydim, terbacil, terbucarb, terbumeton, terbuthylazine, terbutryn, tetflupyrolimet, thaxtomin, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, triclopyr-butotyl, triclopyr-choline, triclopyr-ethyl, triclopyr-triethylammonium, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, 3-(2-chloro-4-fluoro-5-(3-methyl-2,6-dioxo-4-trifluoromethyl-3,6-dihydropyrimidin-1 (2H)-yl)phenyl)-5-methyl-4,5-dihydroisoxazole-5-carboxylic acid ethyl ester, ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetate, 3-chloro-2-[3-(difluoromethyl)isoxazolyl-5-yl]phenyl-5-chloropyrimidin-2-yl ether, 2-(3,4-dimethoxyphenyl)-4-[(2-hydroxy-6-oxocyclohex-1-en-1-yl)carbonyl]-6-methylpyridazine-3(2H)-one, 2-({2-[(2-methoxyethoxy)methyl]-6-methylpyridin-3-yl}carbonyl)cyclohexane-1,3-dione, (5-hydroxy-1-methyl-1H-pyrazol-4-yl)(3,3,4-trimethyl-1,1-dioxido-2,3-dihydro-1-benzothiophen-5-yl)methanone, 1-methyl-4-[(3,3,4-trimethyl-1,1-dioxido-2,3-dihydro-1-benzothiophen-5-yl)carbonyl]-1H-pyrazol-5-yl propane-1-sulfonate, 4-{2-chloro-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazole-4-carboxylate; cyanomethyl 4-amino-3-chloro-5-fluoro-6-(7-fluoro-1H-indol-6-yl)pyridine-2-carboxylate, prop-2-yn-1-yl 4-amino-3-chloro-5-fluoro-6-(7-fluoro-1H-indol-6-yl)pyridine-2-carboxylate, methyl 4-amino-3-chloro-5-fluoro-6-(7-fluoro-1H-indol-6-yl)pyridine-2-carboxylate, 4-amino-3-chloro-5-fluoro-6-(7-fluoro-1H-indol-6-yl)pyridine-2-carboxylic acid, benzyl 4-amino-3-chloro-5-fluoro-6-(7-fluoro-1H-indol-6-yl)pyridine-2-carboxylate, ethyl 4-amino-3-chloro-5-fluoro-6-(7-fluoro-1H-indol-6-yl)pyridine-2-carboxylate, methyl 4-amino-3-chloro-5-fluoro-6-(7-fluoro-1-isobutyryl-1H-indol-6-yl)pyridine-2-carboxylate, methyl 6-(1-acetyl-7-fluoro-1H-indol-6-yl)-4-amino-3-chloro-5-fluoropyridine-2-carboxylate, methyl 4-amino-3-chloro-6-[1-(2,2-dimethylpropanoyl)-7-fluoro-1H-indol-6-yl]-5-fluoropyridine-2-carboxylate, methyl 4-amino-3-chloro-5-fluoro-6-[7-fluoro-1-(methoxyacetyl)-1H-indol-6-yl]pyridine-2-carboxylate, potassium 4-amino-3-chloro-5-fluoro-6-(7-fluoro-1H-indol-6-yl)pyridine-2-carboxylate, sodium 4-amino-3-chloro-5-fluoro-6-(7-fluoro-1H-indol-6-yl)pyridine-2-carboxylate, butyl 4-amino-3-chloro-5-fluoro-6-(7-fluoro-1H-indol-6-yl)pyridine-2-carboxylate, 4-hydroxy-1-methyl-3-[4-(trifluoromethyl)pyridin-2-yl]imidazolidin-2-one, 3-(5-tert-butyl-1,2-oxazol-3-yl)-4-hydroxy-1-methylimidazolidin-2-one, 3-[5-chloro-4-(trifluormethyl)pyridin-2-yl]-4-hydroxy-1-methylimidazolidin-2-one, 4-hydroxy-1-methoxy-5-methyl-3-[4-(trifluormethyl)pyridin-2-yl]imidazolidin-2-one, 6-[(2-hydroxy-6-oxocyclohex-1-en-1-yl)carbonyl]-1,5-dimethyl-3-(2-methylphenyl)quinazolin-2,4(1H,3H)-dione, 3-(2,6-dimethylphenyl)-6-[(2-hydroxy-6-oxocyclohex-1-en-1-yl)carbonyl]-1-methylquinazolin-2,4(1H,3H)-dione, 2-[2-chloro-4-(methylsulfonyl)-3-(morpholin-4-ylmethyl)benzoyl]-3-hydroxycyclohex-2-en-1-one, 1-(2-carboxyethyl)-4-(pyrimidin-2-yl)pyridazin-1-ium salt (with anions such as chloride, acetate or trifluoroacetate), 1-(2-carboxyethyl)-4-(pyridazin-3-yl)pyridazin-1-ium salt (with anions such as chloride, acetate or trifluoroacetate), 4-(pyrimidin-2-yl)-1-(2-sulfoethyl)pyridazin-1-ium salt (with anions such as chloride, acetate or trifluoroacetate), 4-(pyridazin-3-yl)-1-(2-sulfoethyl)pyridazin-1-ium salt (with anions such as chloride, acetate or trifluoroacetate).

Examples of plant growth regulators as possible mixing partners are:

Abscisic acid, acibenzolar, acibenzolar-S-methyl, 1-aminocyclopro-1-yl carboxylic acid and derivatives thereof,5-Aminolävulinsäure, ancymidol, 6-benzylaminopurine, bikinin, brassinolide, brassinolide-ethyl, catechin, chitooligosaccharides (CO; COs differ from LCOs in that they lack the pendant fatty acid chain that is characteristic of LCOs. COs, sometimes referred to as N-acetylchitooligosaccharides, are also composed of GlcNAc residues but have side chain decorations that make them different from chitin molecules [$(C_8H_{13}NO_5)_n$, CAS No. 1398-61-4] and chitosan molecules [$(C_5H_{11}NO_4)_n$, CAS No. 9012-76-4]), chitinous compounds, chlormequat chloride, cloprop, cyclanilide, 3-(Cycloprop-1-enyl)propionic acid, daminozide, dazomet, dazomet-sodium, n-decanol, dikegulac, dikegulac-sodium, endothal, endothal-dipotassium, -disodium, and mono(N,N-dimethylalkylammonium), ethephon, flumetralin, flurenol, flurenol-butyl, flurenol-methyl, flurprimidol, forchlorfenuron, gibberellic acid, inabenfide, indol-3-acetic acid (IAA), 4-indol-3-ylbutyric acid, isoprothiolane, probenazole, jasmonic acid, Jasmonic acid or derivatives thereof (e.g. Jasmonic acid methyl ester), lipo-chitooligosaccharides (LCO, sometimes referred to as symbiotic nodulation (Nod) signals (or Nod factors) or as Myc factors, consist of an oligosaccharide backbone of β-1,4-linked *N*-acetyl-D-glucosamine ("GlcNAc") residues with an N-linked fatty acyl chain condensed at the non-reducing end. As understood in the art, LCOs differ in the number of GlcNAc residues in the backbone, in the length and degree of saturation of the fatty acyl chain and in the substitutions of reducing and non-reducing sugar residues), linoleic acid or derivatives thereof, linolenic acid or derivatives thereof, maleic hydrazide, mepiquat chloride, mepiquat pentaborate, 1-methylcyclopropene, 3'-methyl abscisic acid, 2-(1-naphthyl)acetamide, 1-naphthylacetic acid, 2- naphthyloxyacetic acid, nitrophenolate-mixture, 4-Oxo-4[(2-phenylethyl)amino]butyric acid, paclobutrazol, 4-phenylbutyric acid, N-phenylphthalamic acid, prohexadione, prohexadione-calcium, prohydrojasmon, salicylic acid, salicylic acid methyl ester, strigolacton, tecnazene, thidiazuron, triacontanol, trinexapac, trinexapac-ethyl, tsitodef, uniconazole, uniconazole-P, 2-fluoro-N-(3-methoxyphenyl)-*9H*-purin-6-amine.

Suitable combination partners for the compounds of the general formula (I) according to the invention also include, for example, the following safeners:

S1) Compounds from the group of heterocyclic carboxylic acid derivatives:

S1[a]) Compounds of the dichlorophenylpyrazoline-3-carboxylic acid type (S1[a]), preferably compounds such as 1-(2,4-dichlorophenyl)-5-(ethoxycarbonyl)-5-methyl-2-pyrazoline-3-carboxylic acid, ethyl 1-(2,4-dichlorophenyl)-5-(ethoxycarbonyl)-5-methyl-2-pyrazoline-3-carboxylate (S1-1) ("mefenpyr-diethyl"), and related compounds as described in WO-A-91/07874;

S1[b]) Derivatives of dichlorophenylpyrazolecarboxylic acid (S1[b]), preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4) and related compounds as described in EP-A-333131 131 and EP-A-269806;

S1[c]) Derivatives of 1,5-diphenylpyrazole-3-carboxylic acid (S1[c]), preferably compounds such as ethyl 1-(2,4-dichlorophenyl)-5-phenylpyrazole-3-carboxylate (S1-5), methyl 1-(2-chlorophenyl)-5-phenylpyrazole-3-carboxylate (S1-6) and related compounds as described, for example, in EP-A-268554;

S1[d]) Compounds of the triazolecarboxylic acid type (S1[d]), preferably compounds such as fenchlorazole (ethyl ester), i.e. ethyl 1-(2,4-dichlorophenyl)-5-trichloromethyl-1H-1,2,4-triazole-3-carboxylate (S1-7), and related compounds, as described in EP-A-174562 and EP-A-346620;

S1[e]) Compounds of the 5-benzyl- or 5-phenyl-2-isoxazoline-3-carboxylic acid or of the 5,5-diphenyl-2-isoxazoline-3-carboxylic acid type (S1[e]), preferably compounds such as ethyl 5-(2,4-dichlorobenzyl)-2-isoxazoline-3-carboxylate (S1-8) or ethyl 5-phenyl-2-isoxazoline-3-carboxylate (S1-9) and related compounds as described in WO-A-91/08202, or 5,5-diphenyl-2-isoxazolinecarboxylic acid (S1-10) or ethyl 5,5-diphenyl-2-isoxazoline-3-carboxylate (S1-11) ("isoxadifen-ethyl") or n-propyl 5,5-diphenyl-2-isoxazoline-3-carboxylate (S1-12) or ethyl 5-(4-fluorophenyl)-5-phenyl-2-isoxazoline-3-carboxylate (S1-13), as described in patent application WO-A-95/07897.

S2) Compounds from the group of the 8-quinolinoxy derivatives (S2):

S2[a]) Compounds of the 8-quinolinoxyacetic acid type (S2[a]), preferably 1-methylhexyl (5-chloro-8-quinolinoxy)acetate ("cloquintocet-mexyl") (S2-1), 1,3-dimethylbut-1-yl (5-chloro-8-quinolinoxy)acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl 5-chloro-8-quinolinoxyacetate (S2-6), allyl (5-chloro-8-quinolinoxy)acetate (S2-7), 2-(2-propylideneiminoxy)-1-ethyl (5-chloro-8-quinolinoxy)acetate (S2-8), 2-oxoprop-1-yl (5-chloro-8-quinolinoxy)acetate (S2-9) and related compounds, as described in EP-A-86750, EP-A-94349 and EP-A-191736 or EP-A-0 492 366, and also (5-chloro-8-quinolinoxy)acetic acid (S2-10), hydrates and salts thereof, for example the lithium, sodium, potassium, calcium, magnesium, aluminum, iron, ammonium, quaternary ammonium, sulfonium or phosphonium salts thereof, as described in WO-A-2002/34048;

S2[b]) Compounds of the (5-chloro-8-quinolinoxy)malonic acid type (S2[b]), preferably compounds such as diethyl (5-chloro-8-quinolinoxy)malonate, diallyl (5-chloro-8-quinolinoxy)malonate, methyl ethyl (5-chloro-8-quinolinoxy)malonate and related compounds, as described in EP-A-0 582 198.

S3) Active compounds of the dichloroacetamide type (S3), which are frequently used as pre-emergence safeners

(soil-acting safeners), for example

"dichlormid" (N,N-diallyl-2,2-dichloroacetamide) (S3-1),
"R-29148" (3-dichloroacetyl-2,2,5-trimethyl-1,3-oxazolidine) from Stauffer (S3-2),
"R-28725" (3-dichloroacetyl-2,2-dimethyl-1,3-oxazolidine) from Stauffer (S3-3),
"benoxacor" (4-dichloroacetyl-3,4-dihydro-3-methyl-2H-1,4-benzoxazine) (S3-4),
"PPG-1292" (N-allyl-N-[(1,3-dioxolan-2-yl)methyl]dichloroacetamide) from PPG Industries (S3-5),
"DKA-24" (N-allyl-N-[(allylaminocarbonyl)methyl]dichloroacetamide) from Sagro-Chem (S3-6),
"AD-67" or "MON 4660" (3-dichloroacetyl-1-oxa-3-azaspiro[4.5]decane) from Nitrokemia or Monsanto (S3-7),
"TI-35" (1-dichloroacetylazepane) from TRI-Chemical RT (S3-8),
"Diclonon" (Dicyclonon) or "BAS145138" or "LAB145138" (S3-9)
((RS)-1-dichloroacetyl-3,3,8a-trimethylperhydropyrrolo[1,2-a]pyrimidin-6-one) from BASF, "furilazole" or "MON 13900" ((RS)-3-dichloroacetyl-5-(2-furyl)-2,2-dimethyloxazolidine) (S3-10), and the (R) isomer thereof (S3-11).

S4) Compounds from the class of the acylsulfonamides (S4):

$S4^a$) N-Acylsulfonamides of the formula ($S4^a$) and salts thereof, as described in WO-A-97/45016,

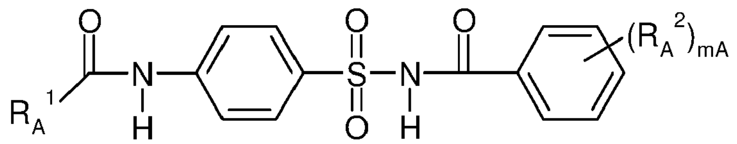

**($S4^a$)**

in which

$R_A^1$ is ($C_1$-$C_6$)-alkyl, ($C_3$-$C_6$)-cycloalkyl, where the 2 latter radicals are substituted by $v_A$ substituents from the group of halogen, ($C_1$-$C_4$)-alkoxy, ($C_1$-$C_6$)-haloalkoxy and ($C_1$-$C_4$)-alkylthio and, in the case of cyclic radicals, also by ($C_1$-$C_4$)-alkyl and ($C_1$-$C_4$)-haloalkyl;

$R_A^2$ is halogen, ($C_1$-$C_4$)-alkyl, ($C_1$-$C_4$)-alkoxy, $CF_3$;

$m_A$ is 1 or 2;

$v_A$ is 0, 1, 2 or 3;

$S4^b$) Compounds of the 4-(benzoylsulfamoyl)benzamide type of the formula ($S4^b$) and salts thereof, as described in WO-A-99/16744,

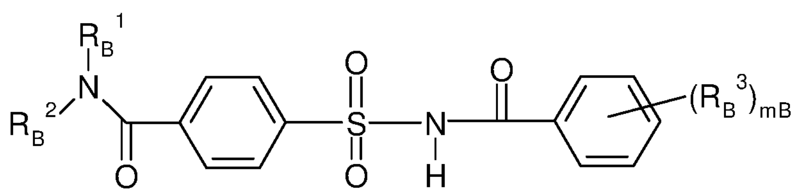

**($S4^b$)**

in which

$R_B^1$, $R_B^2$ are independently hydrogen, ($C_1$-$C_6$)-alkyl, ($C_3$-$C_6$)-cycloalkyl, ($C_3$-$C_6$)-alkenyl, ($C_3$-$C_6$)-alkynyl,
$R_B^3$ is halogen, ($C_1$-$C_4$)-alkyl, ($C_1$-$C_4$)-haloalkyl or ($C_1$-$C_4$)-alkoxy and
$m_B$ is 1 or 2,

for example those in which

$R_B^1$ = cyclopropyl, $R_B^2$ = hydrogen and ($R_B^3$) = 2-OMe ("cyprosulfamide", S4-1),
$R_B^1$ = cyclopropyl, $R_B^2$ = hydrogen and ($R_B^3$) = 5-Cl-2-OMe (S4-2),

$R_B^1$ = ethyl, $R_B^2$ = hydrogen and ($R_B^3$) = 2-OMe (S4-3),
$R_B^1$ = isopropyl, $R_B^2$= hydrogen and ($R_B^3$) = 5-Cl-2-OMe (S4-4) and
$R_B^1$ = isopropyl, $R_B^2$= hydrogen and ($R_B^3$) = 2-OMe (S4-5);

S4$^c$) Compounds from the class of the benzoylsulfamoylphenylureas of the formula (S4$^c$), as described in EP-A-365484,

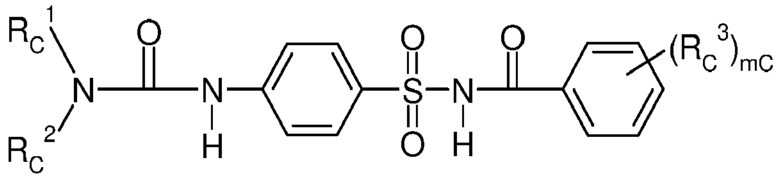

**(S4$^c$)**

in which

$R_C^1$, $R_C^2$ are independently hydrogen, ($C_1$-$C_8$)-alkyl, ($C_3$-$C_8$)-cycloalkyl, ($C_3$-$C_6$)-alkenyl, ($C_3$-$C_6$)-alkynyl,
$R_C^3$ is halogen, ($C_1$-$C_4$)-alkyl, ($C_1$-$C_4$)-alkoxy, $CF_3$ and
$m_C$ is 1 or 2;

for example
1-[4-(N-2-methoxybenzoylsulfamoyl)phenyl]-3-methylurea,

1-[4-(N-2-methoxybenzoylsulfamoyl)phenyl]-3,3-dimethylurea,
1-[4-(N-4,5-dimethylbenzoylsulfamoyl)phenyl]-3-methylurea;

S4$^d$) Compounds of the N-phenylsulfonylterephthalamide type of the formula (S4$^d$) and salts thereof, which are known, for example, from CN 101838227,

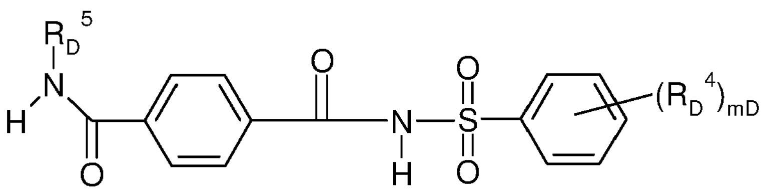

**(S4$^d$)**

in which

$R_D^4$ is halogen, ($C_1$-$C_4$)-alkyl, ($C_1$-$C_4$)-alkoxy, $CF_3$;

$m_D$ is 1 or 2;

$R_D^5$ is hydrogen, ($C_1$-$C_6$)-alkyl, ($C_3$-$C_6$)-cycloalkyl, ($C_2$-$C_6$)-alkenyl, ($C_2$-$C_6$)-alkynyl or ($C_5$-$C_6$)-cycloalkenyl.

S5) Active compounds from the class of the hydroxyaromatics and the aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 2,4-dichlorocinnamic acid, as described in WO-A-2004/084631, WO-A-2005/015994, WO-A-2005/016001.

S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-l,2-dihydroquinoxalin-2-one hydrochloride, 1-(2-methylsulfonylaminoethyl)-3-(2-thienyl)-1,2-dihydroquinoxalin-2-one, as described in WO-A-2005/112630.

S7) Compounds from the class of the diphenylmethoxyacetic acid derivatives (S7), e.g. methyl diphenylmethoxyacetate (CAS Reg. No. 41858-19-9) (S7-1), ethyl diphenylmethoxyacetate or diphenylmethoxyacetic acid, as described in WO-A-98/38856.

S8) Compounds of the formula (S8), as described in WO-A-98/27049,

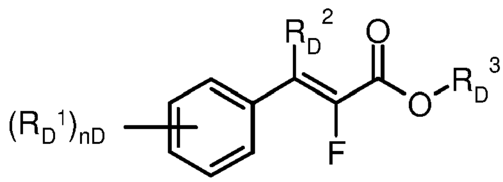

**(S8)**

in which the symbols and indices are defined as follows:

$R_D^1$ is halogen, $(C_1-C_4)$-alkyl, $(C_1-C_4)$-haloalkyl, $(C_1-C_4)$-alkoxy, $(C_1-C_4)$-haloalkoxy,
$R_D^2$ is hydrogen or $(C_1-C_4)$-alkyl,
$R_D^3$ is hydrogen, $(C_1-C_8)$-alkyl, $(C_2-C_4)$-alkenyl, $(C_2-C_4)$-alkynyl or aryl, where each of the aforementioned carbon-containing radicals is unsubstituted or substituted by one or more, preferably up to three identical or different radicals from the group consisting of halogen and alkoxy; or salts thereof,
$n_D$ is an integer from 0 to 2.

S9) active compounds from the class of the 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No. 219479-18-2), 1,2-dihydro-4-hydroxy-1-methyl-3-(5-tetrazolylcarbonyl)-2-quinolone (CAS Reg. No. 95855-00-8), as described in WO-A-199/000020;

S10) Compounds of the formula ($S10^a$) or ($S10^b$)
as described in WO-A-2007/023719 and WO-A-2007/023764

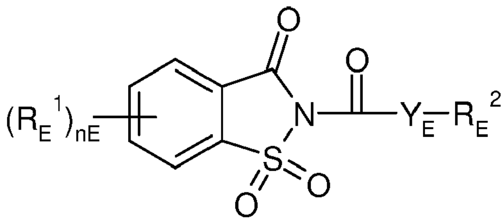

**($S10^a$)**

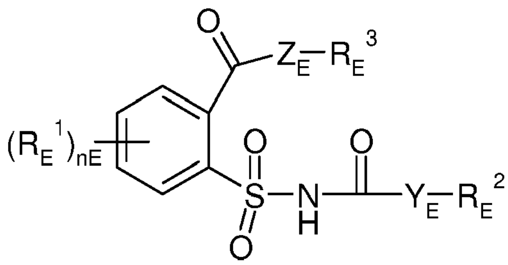

**($S10^b$)**

in which

$R_E^1$ is halogen, $(C_1-C_4)$-alkyl, methoxy, nitro, cyano, $CF_3$, $OCF_3$

$Y_E$, $Z_E$ are independently O or S,

$n_E$ is an integer from 0 to 4,

$R_E^2$ is $(C_1-C_{16})$-alkyl, $(C_2-C_6)$-alkenyl, $(C_3-C_6)$-cycloalkyl, aryl; benzyl, halobenzyl,

$R_E^3$ is hydrogen or $(C_1-C_6)$-alkyl.

S11) Active compounds of the oxyimino compound type (S11), which are known as seed-dressing agents, for example

"oxabetrinil" ((Z)-1,3-dioxolan-2-ylmethoxyimino(phenyl)acetonitrile) (S11-1), which is known as a seed-dressing safener for millet/sorghum against metolachlor damage,

"fluxofenim" (1-(4-chlorophenyl)-2,2,2-trifluoro-1-ethanone O-(1,3-dioxolan-2-ylmethyl)oxime) (S11-2), which is known as a seed-dressing safener for millet/sorghum against metolachlor damage, and

"cyometrinil" or "CGA-43089" ((Z)-cyanomethoxyimino(phenyl)acetonitrile) (S11-3), which is known as a seed-dressing safener for millet/sorghum against metolachlor damage.

S12) active compounds from the class of the isothiochromanones (S12), for example methyl [(3-oxo-1H-2-benzothiopyran-4(3H)-ylidene)methoxy]acetate (CAS Reg. No. 205121-04-6) (S12-1) and related compounds from WO-A-1998/13361.

S13) One or more compounds from group (S13):

"naphthalic anhydride" (1,8-naphthalenedicarboxylic anhydride) (S13-1), which is known as a seed-dressing safener for corn against thiocarbamate herbicide damage,

"fenclorim" (4,6-dichloro-2-phenylpyrimidine) (S13-2), which is known as a safener for pretilachlor in sown rice,

"flurazole" (benzyl 2-chloro-4-trifluoromethyl-1,3-thiazole-5-carboxylate) (S13-3), which is known as a seed-dressing safener for millet/sorghum against alachlor and metolachlor damage,

"CL 304415" (CAS Reg. No. 31541-57-8)

(4-carboxy-3,4-dihydro-2H-1-benzopyran-4-acetic acid) (S13-4) from American Cyanamid, which is known as a safener for corn against damage by imidazolinones,

"MG 191" (CAS Reg. No. 96420-72-3) (2-dichloromethyl-2-methyl-1,3-dioxolane) (S13-5) from Nitrokemia, which is known as a safener for corn,

"MG 838" (CAS Reg. No. 133993-74-5)

(2-propenyl 1-oxa-4-azaspiro[4.5]decane-4-carbodithioate) (S13-6) from Nitrokemia

"disulfoton" (O,O-diethyl S-2-ethylthioethyl phosphorodithioate) (S13-7),

"dietholate" (O,O-diethyl O-phenyl phosphorothioate) (S13-8),

"mephenate" (4-chlorophenyl methylcarbamate) (S13-9).

S14) active compounds which, in addition to herbicidal action against weeds, also have safener action on crop plants such as rice, for example

"dimepiperate" or "MY-93" (*S*-1-methyl 1-phenylethylpiperidine-1-carbothioate), which is known as a safener for rice against damage by the herbicide molinate,

"daimuron" or "SK 23" (1-(1-methyl-1-phenylethyl)-3-p-tolylurea), which is known as safener for rice against imazosulfuron herbicide damage,

"cumyluron" = "JC-940" (3-(2-chlorophenylmethyl)-1-(1-methyl-1-phenylethyl)urea, see JP-A-60087254), which is known as safener for rice against damage by some herbicides,

"methoxyphenone" or "NK 049" (3,3'-dimethyl-4-methoxybenzophenone), which is known as a safener for rice against damage by some herbicides,

"CSB" (1-bromo-4-(chloromethylsulfonyl)benzene) from Kumiai, (CAS Reg. No. 54091-06-4), which is known as a safener against damage by some herbicides in rice.

S15) Compounds of the formula (S15) or tautomers thereof

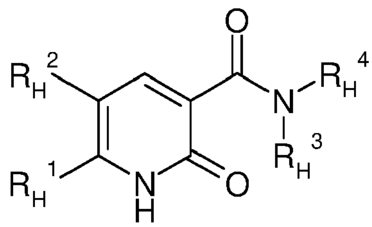

**(S15)**

as described in WO-A-2008/131861 and WO-A-2008/131860
in which

$R_H^1$ is a $(C_1-C_6)$-haloalkyl radical and

$R_H^2$ is hydrogen or halogen and

$R_H^3$, $R_H^4$ are each independently hydrogen, $(C_1-C_{16})$-alkyl, $(C_2-C_{16})$-alkenyl or $(C_2-C_{16})$-alkynyl, where each of the 3 latter radicals is unsubstituted or substituted by one or more radicals from the group of halogen, hydroxyl, cyano, $(C_1-C_4)$-alkoxy, $(C_1-C_4)$-haloalkoxy, $(C_1-C_4)$-alkylthio, $(C_1-C_4)$-alkylamino, di[$(C_1-C_4)$-alkyl]-amino, [$(C_1-C_4)$-alkoxy]-carbonyl, [$(C_1-C_4)$-haloalkoxy]-carbonyl, $(C_3-C_6)$-cycloalkyl which is unsubstituted or substituted, phenyl which is unsubstituted or substituted, and heterocyclyl which is unsubstituted or substituted,

or $(C_3-C_6)$-cycloalkyl, $(C_4-C_6)$-cycloalkenyl, $(C_3-C_6)$-cycloalkyl fused on one side of the ring to a 4 to 6-membered saturated or unsaturated carbocyclic ring, or $(C_4-C_6)$-cycloalkenyl fused on one side of the ring to a 4 to 6-membered saturated or unsaturated carbocyclic ring, where each of the 4 latter radicals is unsubstituted or substituted by one or more radicals from the group of halogen, hydroxyl, cyano, $(C_1-C_4)$-alkyl, $(C_1-C_4)$-haloalkyl, $(C_1-C_4)$-alkoxy, $(C_1-C_4)$-haloalkoxy, $(C_1-C_4)$-alkylthio, $(C_1-C_4)$-alkylamino, di[$(C_1-C_4)$-alkyl]amino, [$(C_1-C_4)$-alkoxy]carbonyl, [$(C_1-C_4)$-haloalkoxy]carbonyl, $(C_3-C_6)$-cycloalkyl which is unsubstituted or substituted, phenyl which is unsubstituted or substituted, and heterocyclyl which is unsubstituted or substituted,

or

$R_H^3$ is $(C_1-C_4)$-alkoxy, $(C_2-C_4)$-alkenyloxy, $(C_2-C_6)$-alkynyloxy or $(C_2-C_4)$-haloalkoxy and

$R_H^4$ is hydrogen or $(C_1-C_4)$-alkyl or

$R_H^3$ and $R_H^4$ together with the directly bonded nitrogen atom are a four- to eight-membered heterocyclic ring which, as well as the nitrogen atom, may also contain further ring heteroatoms, preferably up to two further ring heteroatoms from the group of N, O and S, and which is unsubstituted or substituted by one or more radicals from the group of halogen, cyano, nitro, $(C_1-C_4)$-alkyl, $(C_1-C_4)$-haloalkyl, $(C_1-C_4)$-alkoxy, $(C_1-C_4)$-haloalkoxy and $(C_1-C_4)$-alkylthio.

S16) Active compounds which are used primarily as herbicides but also have safener action on crop plants, for example

(2,4-dichlorophenoxy)acetic acid (2,4-D),

(4-chlorophenoxy)acetic acid,

(R,S)-2-(4-chloro-o-tolyloxy)propionic acid (mecoprop),

4-(2,4-dichlorophenoxy)butyric acid (2,4-DB),

(4-chloro-o-tolyloxy) acetic acid (MCPA),

4-(4-chloro-o-tolyloxy)butyric acid,

4-(4-chlorophenoxy)butyric acid,

3,6-dichloro-2-methoxybenzoic acid (dicamba),

1-(ethoxycarbonyl)ethyl 3,6-dichloro-2-methoxybenzoate (lactidichlor-ethyl).

Preferred safeners in combination with the compounds of the general formula (I) according to the invention and/or their salts and N-oxides, in particular with the compounds of the formulae (1-001) to (I-042), salts or N-oxides thereof, are: cloquintocet-mexyl, cyprosulfamide, fenchlorazole-ethyl, isoxadifen-ethyl, mefenpyr-diethyl, fenclorim, cumyluron, S4-1 and S4-5, and particularly preferred safeners are: cloquintocet-mexyl, cyprosulfamide, isoxadifen-ethyl and mefenpyr-diethyl.

Biological examples:

The following abbreviations are used in the examples and tables below:

Tested harmful plants:

ABUTH: Abutilon theophrasti
ALOMY: Alopecurus myosuroides
AMARE: Amaranthus retroflexus
DIGSA: Digitaria sanguinalis
ECHCG: Echinochloa crus-galli
KCHSC: Kochia scoparia
LOLRI: Lolium rigidum
MATIN: Matricaria inodora
POAAN: Poa annua
SETVI: Setaria viridis
STEME: Stellaria media
VERPE: Veronica persica

A. Herbicidal pre-emergence action

Seeds of mono- and dicotyledonous weed plants were sown in plastic pots (double sowings with one species of mono- and one species of dicotyledonous weed plants per pot), in sandy loam, and covered with soil. The compounds according to the invention, formulated in the form of wettable powders (WP) or as emulsifiable concentrates (EC), were applied to the surface of the covering soil as an aqueous suspension or as an emulsion, with the addition of 0.5% of an additive, at an application rate of 600 litres of water per hectare (converted). Following treatment, the pots were placed in a greenhouse and kept under optimum growth conditions for the test plants. The visual grading of the damage to the test plants is carried out after ca. 3 weeks in comparison to untreated controls (herbicidal effect in percent (%): 100% effect = plants have died, 0% effect = as control plants).

Tables A1 to A12, below, show the effects of selected compounds of the general formula (I) according to Table 1 on various harmful plants and an application rate corresponding to 1280 g/ha obtained by the experimental procedure mentioned above.

Table A1

| Example Number | Dosage [g/ha] | ABUTH |
|---|---|---|
| I-013 | 1280 | 90 |
| 1-020 | 1280 | 100 |

Table A2

| Example Number | Dosage [g/ha] | ALOMY |
|---|---|---|
| I-011 | 1280 | 90 |
| I-013 | 1280 | 90 |
| I-015 | 1280 | 100 |

Table A3

| Example Number | Dosage [g/ha] | ECHCG |
|---|---|---|
| I-013 | 1280 | 100 |

Table A4

| Example Number | Dosage [g/ha] | KCHSC |
|---|---|---|
| 1-004 | 1280 | 90 |
| I-013 | 1280 | 100 |
| I-016 | 1280 | 90 |

Table A5

| Example Number | Dosage [g/ha] | LOLRI |
|---|---|---|
| 1-002 | 1280 | 90 |
| 1-004 | 1280 | 100 |
| I-007 | 1280 | 100 |
| I-011 | 1280 | 90 |
| 1-012 | 1280 | 100 |
| I-013 | 1280 | 100 |
| I-015 | 1280 | 90 |
| 1-023 | 1280 | 90 |
| 1-026 | 1280 | 90 |
| I-027 | 1280 | 100 |

Table A6

| Example Number | Dosage [g/ha] | MATIN |
|---|---|---|
| 1-002 | 1280 | 90 |
| 1-004 | 1280 | 90 |
| I-007 | 1280 | 100 |
| I-011 | 1280 | 100 |
| I-013 | 1280 | 100 |
| I-015 | 1280 | 100 |
| I-016 | 1280 | 90 |
| I-018 | 1280 | 100 |
| 1-023 | 1280 | 100 |
| 1-026 | 1280 | 90 |
| I-027 | 1280 | 90 |
| 1-031 | 1280 | 90 |

Table A7

| Example Number | Dosage [g/ha] | SETVI |
|---|---|---|
| 1-002 | 1280 | 90 |
| 1-004 | 1280 | 90 |
| I-007 | 1280 | 90 |
| 1-030 | 1280 | 100 |

Table A8

| Example Number | Dosage [g/ha] | STEME |
|---|---|---|
| 1-002 | 1280 | 100 |
| 1-004 | 1280 | 90 |
| I-007 | 1280 | 100 |
| I-011 | 1280 | 90 |
| I-013 | 1280 | 100 |
| I-015 | 1280 | 90 |
| I-018 | 1280 | 100 |
| 1-023 | 1280 | 100 |
| 1-024 | 1280 | 90 |
| 1-026 | 1280 | 90 |
| I-027 | 1280 | 100 |

Table A9

| Example Number | Dosage [g/ha] | VERPE |
|---|---|---|
| 1-004 | 1280 | 90 |
| I-013 | 1280 | 100 |
| I-015 | 1280 | 100 |
| I-018 | 1280 | 100 |

Table A10

| Example Number | Dosage [g/ha] | POAAN |
|---|---|---|
| 1-002 | 1280 | 100 |
| 1-004 | 1280 | 90 |
| I-007 | 1280 | 100 |
| I-011 | 1280 | 100 |
| 1-012 | 1280 | 90 |
| I-013 | 1280 | 90 |
| I-015 | 1280 | 100 |
| 1-026 | 1280 | 90 |

(continued)

| Example Number | Dosage [g/ha] | POAAN |
|---|---|---|
| I-027 | 1280 | 90 |

Table A11

| Example Number | Dosage [g/ha] | AMARE |
|---|---|---|
| 1-002 | 1280 | 100 |
| 1-004 | 1280 | 90 |
| I-007 | 1280 | 100 |
| I-011 | 1280 | 90 |
| I-013 | 1280 | 100 |
| 1-023 | 1280 | 100 |
| 1-026 | 1280 | 90 |
| I-038 | 1280 | 90 |

Table A12

| Example Number | Dosage [g/ha] | DIGSA |
|---|---|---|
| 1-004 | 1280 | 90 |
| I-015 | 1280 | 100 |

As the results show, various compounds of the general formula (I) according to the invention have very good herbicidal pre-emergence efficacy against a broad spectrum of harmful mono- and dicotyledonous plants such as Abutilon theophrasti (ABUTH), Alopecurus myosuroides (ALOMY), Amaranthus retroflexus (AMARE), Digitaria sanguinalis (DIGSA), Echinochloa crus-galli (ECHCG), Kochia scoparia (KCHSC), Lolium rigidum (LOLRI), Matricaria inodora (MATIN), Poa annua (POAAN), Setaria viridis (SETVI), Stellaria media (STEME) and Veronica persica (VERPE) at an application rate of 1280 g of active ingredient per hectare.

B. Herbicidal post-emergence action

Seeds of mono- and dicotyledonous weed plants were sown in plastic pots (double sowings with one species of mono- and one species of dicotyledonous weed plants per pot), in sandy loam, covered with soil and grown under controlled growth conditions. 2 to 3 weeks after sowing, the test plants were sprayed in the single-leaf stage. The compounds according to the invention, formulated in the form of wettable powders (WP) or as emulsifiable concentrates (EC), were sprayed onto the green plant parts as an aqueous suspension or as an emulsion, with the addition of 0.5% of an additive, at an application rate of 600 litres of water per hectare (converted). The test plants were placed in the greenhouse for ca. 3 weeks under optimum growth conditions, and then the effect of the preparations is assessed visually in comparison with untreated controls (herbicidal effect in percent (%): 100% effect = plants have died, 0% effect = as control plants).

Tables B1 to B11, below, show the effects of selected compounds of the general formula (I) according to Table 1 on various harmful plants using an application rate corresponding to 1280 g/ha obtained by the experimental procedure mentioned above.

Table B1

| Example Number | Dosage [g/ha] | ABUTH |
|---|---|---|
| I-011 | 1280 | 90 |
| 1-012 | 1280 | 90 |

(continued)

| Example Number | Dosage [g/ha] | ABUTH |
|---|---|---|
| I-015 | 1280 | 100 |
| I-018 | 1280 | 100 |

Table B2

| Example Number | Dosage [g/ha] | ALOMY |
|---|---|---|
| 1-004 | 1280 | 100 |
| I-011 | 1280 | 100 |
| I-013 | 1280 | 90 |
| I-015 | 1280 | 100 |
| I-027 | 1280 | 90 |

Table B3

| Example Number | Dosage [g/ha] | KCHSC |
|---|---|---|
| 1-004 | 1280 | 90 |
| 1-006 | 1280 | 90 |
| I-007 | 1280 | 90 |
| I-011 | 1280 | 100 |
| 1-012 | 1280 | 90 |
| I-013 | 1280 | 100 |
| I-015 | 1280 | 100 |
| I-016 | 1280 | 100 |
| I-018 | 1280 | 90 |
| 1-022 | 1280 | 90 |
| 1-023 | 1280 | 90 |
| 1-024 | 1280 | 90 |
| 1-026 | 1280 | 90 |
| I-027 | 1280 | 90 |
| I-029 | 1280 | 90 |
| 1-031 | 1280 | 90 |
| 1-032 | 1280 | 100 |

Table B4

| Example Number | Dosage [g/ha] | LOLRI |
|---|---|---|
| 1-004 | 1280 | 100 |
| I-007 | 1280 | 90 |
| I-011 | 1280 | 90 |
| I-013 | 1280 | 100 |

(continued)

| Example Number | Dosage [g/ha] | LOLRI |
|---|---|---|
| I-015 | 1280 | 100 |

Table B5

| Example Number | Dosage [g/ha] | MATIN |
|---|---|---|
| 1-002 | 1280 | 100 |
| 1-004 | 1280 | 100 |
| 1-006 | 1280 | 100 |
| I-007 | 1280 | 100 |
| I-011 | 1280 | 100 |
| 1-012 | 1280 | 100 |
| I-013 | 1280 | 100 |
| I-015 | 1280 | 100 |
| I-016 | 1280 | 100 |
| 1-017 | 1280 | 90 |
| I-018 | 1280 | 100 |
| 1-021 | 1280 | 90 |
| 1-022 | 1280 | 100 |
| 1-023 | 1280 | 100 |
| 1-024 | 1280 | 90 |
| 1-025 | 1280 | 90 |
| I-027 | 1280 | 90 |
| I-029 | 1280 | 90 |
| 1-030 | 1280 | 100 |
| 1-031 | 1280 | 90 |
| 1-032 | 1280 | 90 |

Table B6

| Example Number | Dosage [g/ha] | SETVI |
|---|---|---|
| 1-004 | 1280 | 90 |
| 1-012 | 1280 | 90 |
| I-016 | 1280 | 90 |
| I-027 | 1280 | 90 |
| 1-030 | 1280 | 90 |
| 1-032 | 1280 | 90 |
| 1-040 | 1280 | 90 |

Table B7

| Example Number | Dosage [g/ha] | STEME |
|---|---|---|
| 1-002 | 1280 | 100 |
| 1-004 | 1280 | 100 |
| 1-006 | 1280 | 90 |
| I-007 | 1280 | 100 |
| I-011 | 1280 | 100 |
| 1-012 | 1280 | 100 |
| I-013 | 1280 | 100 |
| I-015 | 1280 | 100 |
| I-016 | 1280 | 100 |
| I-018 | 1280 | 90 |
| 1-022 | 1280 | 90 |
| 1-023 | 1280 | 90 |
| 1-024 | 1280 | 90 |
| 1-026 | 1280 | 90 |
| I-027 | 1280 | 90 |
| I-038 | 1280 | 90 |

Table B8

| Example Number | Dosage [g/ha] | VERPE |
|---|---|---|
| 1-004 | 1280 | 100 |
| 1-006 | 1280 | 90 |
| I-011 | 1280 | 100 |
| 1-012 | 1280 | 100 |
| I-013 | 1280 | 100 |
| I-015 | 1280 | 100 |
| I-016 | 1280 | 90 |
| I-018 | 1280 | 90 |
| 1-021 | 1280 | 90 |
| 1-022 | 1280 | 100 |
| 1-025 | 1280 | 90 |
| 1-026 | 1280 | 90 |
| I-027 | 1280 | 90 |
| 1-030 | 1280 | 90 |
| 1-031 | 1280 | 90 |
| 1-032 | 1280 | 100 |
| I-038 | 1280 | 90 |
| I-039 | 1280 | 90 |
| 1-040 | 1280 | 90 |

Table B9

| Example Number | Dosage [g/ha] | POAAN |
|---|---|---|
| 1-002 | 1280 | 100 |
| 1-004 | 1280 | 100 |
| I-007 | 1280 | 100 |
| I-011 | 1280 | 90 |
| 1-012 | 1280 | 90 |
| I-013 | 1280 | 100 |
| I-015 | 1280 | 100 |
| I-016 | 1280 | 100 |
| 1-017 | 1280 | 100 |
| I-018 | 1280 | 90 |
| 1-023 | 1280 | 90 |
| 1-026 | 1280 | 90 |
| I-027 | 1280 | 90 |

Table B10

| Example Number | Dosage [g/ha] | AMARE |
|---|---|---|
| 1-002 | 1280 | 100 |
| 1-004 | 1280 | 100 |
| 1-006 | 1280 | 100 |
| I-011 | 1280 | 100 |
| 1-012 | 1280 | 100 |
| I-013 | 1280 | 100 |
| I-015 | 1280 | 90 |
| I-016 | 1280 | 100 |
| 1-017 | 1280 | 100 |
| I-018 | 1280 | 100 |
| I-019 | 1280 | 100 |
| 1-020 | 1280 | 90 |
| 1-021 | 1280 | 100 |
| 1-022 | 1280 | 100 |
| 1-023 | 1280 | 90 |
| 1-024 | 1280 | 100 |
| 1-025 | 1280 | 100 |
| 1-026 | 1280 | 100 |
| I-027 | 1280 | 90 |
| 1-030 | 1280 | 90 |
| 1-031 | 1280 | 90 |

(continued)

| Example Number | Dosage [g/ha] | AMARE |
|---|---|---|
| 1-032 | 1280 | 90 |
| I-038 | 1280 | 100 |
| I-039 | 1280 | 90 |
| 1-040 | 1280 | 100 |

Table B11

| Example Number | Dosage [g/ha] | DIGSA |
|---|---|---|
| 1-004 | 1280 | 100 |
| I-011 | 1280 | 100 |
| I-015 | 1280 | 100 |

As the results show, various compounds of the general formula (I) according to the invention have very good herbicidal post-emergence efficacy against a broad spectrum of harmful mono- and dicotyledonous plants such as Abutilon theophrasti (ABUTH), Alopecurus myosuroides (ALOMY), Amaranthus retroflexus (AMARE), Digitaria sanguinalis (DIGSA), Kochia scoparia (KCHSC), Lolium rigidum (LOLRI), Matricaria inodora (MATIN), Poa annua (POAAN), Setaria viridis (SETVI), Stellaria media (STEME) and Veronica persica (VERPE) at an application rate of 1280 g of active ingredient per hectare.

C. Herbicidal pre-emergence action

Seeds of mono- and dicotyledonous weed plants were sown in plastic pots (double sowings with one species of mono- and one species of dicotyledonous weed plants per pot), in sandy loam, and covered with soil. The compounds according to the invention, formulated in the form of wettable powders (WP) or as emulsifiable concentrates (EC), were applied to the surface of the covering soil as an aqueous suspension or as an emulsion, with the addition of 0.5% of an additive, at an application rate of 600 litres of water per hectare (converted). Following treatment, the pots were placed in a greenhouse and kept under optimum growth conditions for the test plants. The visual grading of the damage to the test plants is carried out after ca. 3 weeks in comparison to untreated controls (herbicidal effect in percent (%): 100% effect = plants have died, 0% effect = as control plants).

Tables C1 to C6 below show the effects of selected compounds of the general formula (I) according to Table 1 on various harmful plants using an application rate corresponding to 320 g/ha obtained by the experimental procedure mentioned above.

Table C1

| Example Number | Dosage [g/ha] | ABUTH |
|---|---|---|
| 1-020 | 320 | 100 |

Table C2

| Example Number | Dosage [g/ha] | KCHSC |
|---|---|---|
| I-016 | 320 | 90 |

Table C3

| Example Number | Dosage [g/ha] | LOLRI |
|---|---|---|
| 1-002 | 320 | 90 |

(continued)

| Example Number | Dosage [g/ha] | LOLRI |
|---|---|---|
| I-013 | 320 | 90 |
| I-027 | 320 | 90 |

Table C4

| Example Number | Dosage [g/ha] | MATIN |
|---|---|---|
| I-018 | 320 | 90 |

Table C5

| Example Number | Dosage [g/ha] | STEME |
|---|---|---|
| 1-002 | 320 | 90 |
| 1-004 | 320 | 90 |
| I-007 | 320 | 100 |
| 1-026 | 320 | 90 |

Table C6

| Example Number | Dosage [g/ha] | POAAN |
|---|---|---|
| 1-002 | 320 | 100 |
| 1-004 | 320 | 90 |
| I-007 | 320 | 100 |
| I-015 | 320 | 100 |
| I-027 | 320 | 90 |

As the results show, various compounds of the general formula (I) according to the invention have very good herbicidal pre-emergence efficacy against a broad spectrum of harmful mono- and dicotyledonous plants such as Abutilon theophrasti (ABUTH), Kochia scoparia (KCHSC), Lolium rigidum (LOLRI), Matricaria inodora (MATIN), Poa annua (POAAN) and Stellaria media (STEME) at an application rate of 320 g of active ingredient per hectare.

D. Herbicidal post-emergence action

Seeds of mono- and dicotyledonous weed plants were sown in plastic pots (double sowings with one species of mono- and one species of dicotyledonous weed plants per pot), in sandy loam, covered with soil and grown under controlled growth conditions. 2 to 3 weeks after sowing, the test plants were sprayed in the single-leaf stage. The compounds according to the invention, formulated in the form of wettable powders (WP) or as emulsifiable concentrates (EC), are sprayed onto the green plant parts as an aqueous suspension or as an emulsion, with the addition of 0.5% of an additive, at an application rate of 600 litres of water per hectare (converted). The test plants were placed in the greenhouse for ca. 3 weeks under optimum growth conditions, and then the effect of the preparations is assessed visually in comparison with untreated controls

(herbicidal effect in percent (%): 100% effect = plants have died, 0% effect = as control plants).

Tables D1 to D9 below show the effects of selected compounds of the general formula (I) according to Table 1 on various harmful plants using an application rate corresponding to 320 g/ha obtained by the experimental procedure

mentioned above.

Table D1

| Example Number | Dosage [g/ha] | KCHSC |
|---|---|---|
| I-011 | 320 | 90 |
| I-013 | 320 | 100 |
| I-016 | 320 | 90 |
| 1-032 | 320 | 90 |

Table D2

| Example Number | Dosage [g/ha] | LOLRI |
|---|---|---|
| I-013 | 320 | 90 |

Table D3

| Example Number | Dosage [g/ha] | MATIN |
|---|---|---|
| I-011 | 320 | 100 |
| I-013 | 320 | 100 |
| I-015 | 320 | 100 |
| I-016 | 320 | 100 |
| 1-017 | 320 | 90 |
| I-027 | 320 | 90 |
| 1-031 | 320 | 90 |
| 1-032 | 320 | 90 |

Table D4

| Example Number | Dosage [g/ha] | SETVI |
|---|---|---|
| 1-004 | 320 | 90 |

Table D5

| Example Number | Dosage [g/ha] | STEME |
|---|---|---|
| 1-002 | 320 | 90 |
| 1-004 | 320 | 100 |
| I-007 | 320 | 100 |
| I-011 | 320 | 90 |
| I-013 | 320 | 100 |
| I-015 | 320 | 100 |

Table D6

| Example Number | Dosage [g/ha] | VERPE |
|---|---|---|
| 1-004 | 320 | 100 |
| I-011 | 320 | 90 |
| I-013 | 320 | 100 |
| I-015 | 320 | 100 |
| 1-030 | 320 | 90 |
| I-038 | 320 | 90 |
| 1-040 | 320 | 90 |

Table D7

| Example Number | Dosage [g/ha] | POAAN |
|---|---|---|
| 1-004 | 320 | 100 |
| I-013 | 320 | 100 |
| I-015 | 320 | 100 |

Table D8

| Example Number | Dosage [g/ha] | AMARE |
|---|---|---|
| I-011 | 320 | 90 |
| I-018 | 320 | 90 |
| 1-021 | 320 | 90 |
| 1-025 | 320 | 90 |
| I-038 | 320 | 100 |
| 1-040 | 320 | 90 |

Table D9

| Example Number | Dosage [g/ha] | DIGSA |
|---|---|---|
| 1-004 | 320 | 90 |
| I-011 | 320 | 90 |

As the results show, various compounds of the general formula (I) according to the invention have very good herbicidal post-emergence efficacy against a broad spectrum of harmful mono- and dicotyledonous plants such as Amaranthus retroflexus (AMARE), Digitaria sanguinalis (DIGSA), Kochia scoparia (KCHSC), Lolium rigidum (LOLRI), Matricaria inodora (MATIN), Poa annua (POAAN), Setaria viridis (SETVI), Stellaria media (STEME) and Veronica persica (VERPE) at an application rate of 320 g of active ingredient per hectare.

**Claims**

**1.** A substituted 1,2,4-thiadiazolyl isonicotinamide of the general formula (I), salt or N-oxide thereof

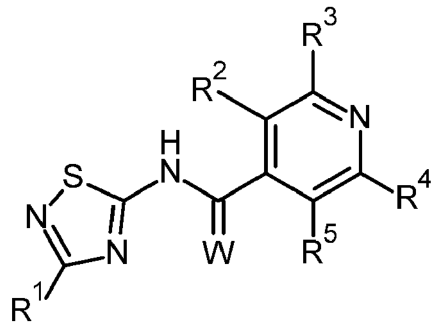

(I)

in which

W represents oxygen or sulfur,
$R^1$ represents hydrogen or halogen,
$R^2$ represents halogen, nitro, ($C_1$-$C_4$)-alkyl, ($C_1$-$C_4$)-haloalkyl, ($C_2$-$C_4$)-alkenyl, ($C_2$-$C_4$)-haloalkenyl, ($C_1$-$C_4$)-haloalkyl-($C_2$-$C_4$)-alkenyl, ($C_1$-$C_4$)-alkoxy, ($C_1$-$C_4$)-haloalkoxy, ($C_1$-$C_4$)-alkylthio, ($C_1$-$C_4$)-haloalkylthio, ($C_1$-$C_4$)-alkylsulfinyl, ($C_1$-$C_4$)-haloalkylsulfinyl, ($C_1$-$C_4$)-alkylsulfonyl, ($C_1$-$C_4$)-haloalkylsulfonyl, ($C_3$-$C_6$)-cycloalkyl, ($C_3$-$C_6$)-halocycloalkyl, ($C_1$-$C_4$)-alkyl-($C_3$-$C_6$)-cycloalkyl, ($C_1$-$C_4$)-alkyl-($C_3$-$C_6$)-halocycloalkyl, ($C_1$-$C_4$)-haloalkyl-($C_3$-$C_6$)-cycloalkyl or ($C_1$-$C_4$)-haloalkyl-($C_3$-$C_6$)-halocycloalkyl,
$R^3$ represents hydrogen, halogen, nitro, cyano, ($C_1$-$C_4$)-alkyl, ($C_1$-$C_4$)-haloalkyl, ($C_1$-$C_4$)-alkoxy, ($C_1$-$C_4$)-haloalkoxy, ($C_3$-$C_6$)-cycloalkyl or ($C_3$-$C_6$)-halocycloalkyl,
$R^4$ represents hydrogen, halogen, nitro, cyano, ($C_1$-$C_4$)-alkyl, ($C_1$-$C_4$)-haloalkyl, ($C_1$-$C_4$)-alkoxy, ($C_1$-$C_4$)-haloalkoxy, ($C_3$-$C_6$)-cycloalkyl or ($C_3$-$C_6$)-halocycloalkyl, and
$R^5$ represents hydrogen, halogen, nitro, cyano, ($C_1$-$C_4$)-alkyl, ($C_1$-$C_4$)-haloalkyl, ($C_1$-$C_4$)-alkoxy, ($C_1$-$C_4$)-haloalkoxy, ($C_3$-$C_6$)-cycloalkyl or ($C_3$-$C_6$)-halocycloalkyl.

**2.** The compound of the general formula (I) as claimed in claim 1 and/or the salt thereof, **characterized in that**

W represents oxygen or sulfur,
$R^1$ represents hydrogen or halogen,
$R^2$ represents halogen, nitro, ($C_1$-$C_4$)-alkyl, ($C_1$-$C_4$)-haloalkyl, ($C_2$-$C_4$)-alkenyl, ($C_2$-$C_4$)-haloalkenyl, ($C_1$-$C_4$)-alkoxy, ($C_1$-$C_4$)-haloalkoxy, ($C_1$-$C_4$)-alkylthio, ($C_1$-$C_4$)-haloalkylthio, ($C_1$-$C_4$)-alkylsulfinyl, ($C_1$-$C_4$)-haloalkylsulfinyl, ($C_1$-$C_4$)-alkylsulfonyl, ($C_1$-$C_4$)-haloalkylsulfonyl, ($C_3$-$C_6$)-cycloalkyl, ($C_3$-$C_6$)-halocycloalkyl, ($C_1$-$C_4$)-alkyl-($C_3$-$C_6$)-cycloalkyl or ($C_1$-$C_4$)-alkyl-($C_3$-$C_6$)-halocycloalkyl,
$R^3$ represents hydrogen, halogen, ($C_1$-$C_4$)-alkyl, ($C_1$-$C_4$)-haloalkyl, ($C_1$-$C_4$)-alkoxy or ($C_1$-$C_4$)-haloalkoxy,
$R^4$ represents hydrogen, halogen, cyano, ($C_1$-$C_4$)-alkyl, ($C_1$-$C_4$)-haloalkyl, ($C_1$-$C_4$)-alkoxy, ($C_1$-$C_4$)-haloalkoxy or ($C_3$-$C_6$)-cycloalkyl, and
$R^5$ represents hydrogen, halogen, ($C_1$-$C_4$)-alkyl, ($C_1$-$C_4$)-haloalkyl, ($C_1$-$C_4$)-alkoxy or ($C_1$-$C_4$)-haloalkoxy.

**3.** The compound of the general formula (I) as claimed in claim 1 and/or the salt thereof, **characterized in that**

W represents oxygen or sulfur, preferably oxygen,
$R^1$ represents hydrogen or halogen,
$R^2$ represents halogen, nitro, ($C_1$-$C_4$)-alkyl, ($C_1$-$C_4$)-haloalkyl, ($C_2$-$C_4$)-alkenyl, ($C_1$-$C_4$)-alkoxy, ($C_1$-$C_4$)-haloalkoxy, ($C_1$-$C_4$)-alkylthio, ($C_1$-$C_4$)-haloalkylthio, ($C_1$-$C_4$)-alkylsulfonyl, ($C_1$-$C_4$)-haloalkylsulfonyl, ($C_3$-$C_6$)-cycloalkyl or ($C_3$-$C_6$)-halocycloalkyl,
$R^3$ represents hydrogen, halogen, ($C_1$-$C_4$)-alkyl or ($C_1$-$C_4$)-haloalkyl,
$R^4$ represents hydrogen, halogen, ($C_1$-$C_4$)-alkyl, ($C_1$-$C_4$)-haloalkyl, ($C_1$-$C_4$)-alkoxy or ($C_1$-$C_4$)-haloalkoxy, and $R^5$ represents hydrogen, halogen, ($C_1$-$C_4$)-alkyl or ($C_1$-$C_4$)-haloalkyl.

**4.** The compound of the general formula (I) as claimed in claim 1 and/or the salt thereof, **characterized in that**

W represents oxygen,
$R^1$ represents hydrogen or halogen,

$R^2$ represents halogen, nitro, ($C_1$-$C_4$)-alkyl, ($C_1$-$C_4$)-haloalkyl, ($C_1$-$C_4$)-haloalkoxy, ($C_1$-$C_4$)-alkylthio, ($C_1$-$C_4$)-haloalkylthio, ($C_1$-$C_4$)-alkylsulfonyl or ($C_3$-$C_6$)-cycloalkyl,
$R^3$ represents hydrogen or halogen,
$R^4$ represents hydrogen, halogen, ($C_1$-$C_4$)-haloalkyl or ($C_1$-$C_4$)-alkoxy,
and $R^5$ represents hydrogen or halogen.

**5.** The compound of the general formula (I) as claimed in claim 1 and/or the salt thereof, **characterized in that**

W represents oxygen,
$R^1$ represents hydrogen, chlorine or bromine,
$R^2$ represents fluorine, chlorine, bromine, iodine, nitro, methyl, trifluoromethyl, trifluoromethoxy, ethylthio, 2,2,2-trifluoroethylthio, ethylsulfonyl or cyclopropyl,
$R^3$ represents hydrogen, fluorine, chlorine or bromine,
$R^4$ represents hydrogen, fluorine, chlorine, bromine, trifluoromethyl or methoxy,
and $R^5$ represents hydrogen, fluorine, chlorine or bromine.

**6.** The compound of the general formula (I) as claimed in claim 1 and/or the salt thereof, **characterized in that**

W represents oxygen,
$R^1$ represents hydrogen, chlorine or bromine,
$R^2$ represents fluorine, chlorine, bromine, iodine, nitro, trifluoromethyl, trifluoromethoxy, ethylsulfonyl or cyclopropyl,
$R^3$ represents hydrogen, fluorine, chlorine or bromine,
$R^4$ represents hydrogen, fluorine, chlorine, bromine or trifluoromethyl,
and $R^5$ represents hydrogen or bromine.

**7.** The use of one or more compounds of the general formula (I) and/or salts thereof, as defined in any of claims 1 to 6, as herbicide and/or plant growth regulator.

**8.** A herbicidal and/or plant growth-regulating composition, **characterized in that** the composition comprises one or more compounds of the formula (I) and/or salts thereof as defined in any of claims 1 to 6, and one or more further substances selected from groups (i) and/or (ii), with

(i) one or more further agrochemically active substances, selected from the group consisting of insecticides, acaricides, nematicides, further herbicides, fungicides, safeners, fertilizers and/or further growth regulators,
(ii) one or more formulation auxiliaries customary in crop protection.

**9.** A method for controlling harmful plants or for regulating the growth of plants, **characterized in that** an effective amount

- of one or more compounds of the formula (I) and/or salts thereof, as defined in any of claims 1 to 6, or
- of a composition as claimed in claim 8, is applied to the plants, seeds of plants, the soil in which or on which the plants grow or the area under cultivation.

Europäisches Patentamt
European Patent Office
Office européen des brevets

# EUROPEAN SEARCH REPORT

Application Number
EP 22 16 0304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2017/005717 A1 (BAYER CROPSCIENCE AG [DE]) 12 January 2017 (2017-01-12)<br>* claim 1 *<br>----- | 1-9 | INV.<br>C07D417/12<br>C07D285/08<br>A01N43/82 |
| A,D | WO 2018/108791 A1 (BAYER CROPSCIENCE AG [DE]; BAYER AG [DE])<br>21 June 2018 (2018-06-21)<br>* claim 1 *<br>----- | 1-9 | |
| | | | TECHNICAL FIELDS SEARCHED (IPC) |
| | | | C07D<br>A01N |

The present search report has been drawn up for all claims

1

| Place of search | Date of completion of the search | Examiner |
|---|---|---|
| Munich | 1 August 2022 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 16 0304

This annex lists the patent family members relating to the patent documents cited in the above-mentioned European search report. The members are as contained in the European Patent Office EDP file on
The European Patent Office is in no way liable for these particulars which are merely given for the purpose of information.

01-08-2022

| Patent document cited in search report | Publication date | Patent family member(s) | Publication date |
|---|---|---|---|
| WO 2017005717 A1 | 12-01-2017 | AR 105278 A1 | 20-09-2017 |
| | | BR 112018000308 A2 | 04-09-2018 |
| | | CN 107846888 A | 27-03-2018 |
| | | JP 6732877 B2 | 29-07-2020 |
| | | JP 2018525352 A | 06-09-2018 |
| | | KR 20180025949 A | 09-03-2018 |
| | | PH 12018500009 A1 | 09-07-2018 |
| | | RU 2018104284 A | 06-08-2019 |
| | | TW 201716389 A | 16-05-2017 |
| | | US 2018213782 A1 | 02-08-2018 |
| | | UY 36776 A | 31-01-2017 |
| | | WO 2017005717 A1 | 12-01-2017 |
| WO 2018108791 A1 | 21-06-2018 | AR 110375 A1 | 20-03-2019 |
| | | BR 112019012269 A2 | 03-12-2019 |
| | | CN 110382493 A | 25-10-2019 |
| | | EP 3555089 A1 | 23-10-2019 |
| | | JP 2020502168 A | 23-01-2020 |
| | | TW 201835060 A | 01-10-2018 |
| | | US 2019382358 A1 | 19-12-2019 |
| | | UY 37527 A | 31-07-2018 |
| | | WO 2018108791 A1 | 21-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4416683 A
- US 4515625 A
- US 4636243 A
- US 4801718 A
- US 4343945 A
- DE 2154852
- DE 19601139
- WO 0136415 A
- WO 0140206 A
- WO 0140223 A
- WO 0146165 A
- WO 2017005717 A
- WO 2018108791 A
- US 20190233382 A
- WO 2015123133 A
- WO 9107874 A
- EP 333131131 A
- EP 269806 A
- EP 268554 A
- EP 174562 A
- EP 346620 A
- WO 9108202 A
- WO 9507897 A
- EP 86750 A
- EP 94349 A
- EP 191736 A
- EP 0492366 A
- WO 200234048 A
- EP 0582198 A
- WO 9745016 A
- WO 9916744 A
- EP 365484 A
- CN 101838227
- WO 2004084631 A
- WO 2005015994 A
- WO 2005016001 A
- WO 2005112630 A
- WO 9838856 A
- WO 9827049 A
- WO 199000020 A
- WO 2007023719 A
- WO 2007023764 A
- WO 199813361 A
- JP 60087254 A
- WO 2008131861 A
- WO 2008131860 A


### Non-patent literature cited in the description


- *Chem. Soc. Rev.,* 2009, vol. 38, 606-631
- *Chem. Commun.,* 2008, 1100-1102
- *Tetrahedron Lett.,* 2006, vol. 47, 5767-5769
- *J. Org. Chem.,* 2011, vol. 76, 8036-8041
- *J. Am. Chem. Soc.,* 2003, vol. 125, 5616-5617
- *Angew. Chem. Int. Ed.,* 2004, vol. 43, 1132-1136
- **BRAUN et al.** *EMBO J,* 1992, vol. 11, 3219-3227
- The Pesticide Manual. The British Crop Protection Council and the Royal Soc. of Chemistry, 2012
- **WATKINS.** Handbook of Insecticide Dust Diluents and Carriers. Darland Books
- **H.V. OLPHEN.** Introduction to Clay Colloid Chemistry. J. Wiley & Sons
- **C. MARSDEN.** Solvents Guide. Interscience, 1963
- **MCCUTCHEON'S.** Detergents and Emulsifiers Annual. MC Publ. Corp,
- **SISLEY ; WOOD.** Encyclopedia of Surface Active Agents. Chem. Publ. Co. Inc, 1964
- **SCHÖNFELDT.** Grenzflachenaktive Äthylenoxidaddukte. Wiss. Verlagsgesellschaft, 1976
- **WINNACKER-KÜCHLER.** Chemische Technologie. C. Hanser Verlag, 1986, vol. 7
- Spray Drying Handbook. G. Goodwin Ltd, 1979
- **J.E. BROWNING.** Agglomeration. *Chemical and Engineering,* 1967, 147
- Perry's Chemical Engineer's Handbook. McGraw Hill, 1973, 8-57
- **G.C. KLINGMAN.** Weed Control as a Science. John Wiley and Sons, Inc, 1961, 81-96
- **J.D. FREYER ; S.A. EVANS.** Weed Control Handbook. Blackwell Scientific Publications, 1968, 101-103
- Chemistry and Technology of Agrochemical Formulations. Kluwer Academic Publishers, 1998
- *Weed Research,* 1986, vol. 26, 441-445
- *CHEMICAL ABSTRACTS,* 1398-61-4

- *CHEMICAL ABSTRACTS,* 9012-76-4
- *CHEMICAL ABSTRACTS,* 41858-19-9
- *CHEMICAL ABSTRACTS,* 219479-18-2
- *CHEMICAL ABSTRACTS,* 95855-00-8
- *CHEMICAL ABSTRACTS,* 205121-04-6
- *CHEMICAL ABSTRACTS,* 31541-57-8
- *CHEMICAL ABSTRACTS,* 96420-72-3
- *CHEMICAL ABSTRACTS,* 133993-74-5
- *CHEMICAL ABSTRACTS,* 54091-06-4